(19) Europäisches Patentamt
European Patent Office
Office européen des brevets

(11) Publication number: **0 203 528 B1**

(12) **EUROPEAN PATENT SPECIFICATION**

(45) Date of publication of the patent specification: **01.08.90**

(51) Int. Cl.⁵: **C07C 33/12,** C07C 49/21, C07C 29/136, A61K 7/46

(21) Application number: **86106956.5**

(22) Date of filing: **22.05.86**

(54) **Cyclopentene derivatives and their use as odorants.**

(30) Priority: **31.05.85 US 740467**
**19.03.86 US 841500**

(43) Date of publication of application:
**03.12.86 Bulletin 86/49**

(45) Publication of the grant of the patent:
**01.08.90 Bulletin 90/31**

(84) Designated Contracting States:
**BE CH DE FR GB IT LI NL**

(56) References cited:
**EP-A- 0 155 591**
**US-A- 4 173 585**

(73) Proprietor: **L. GIVAUDAN & CIE Société Anonyme, CH-1214 Vernier-Genève(CH)**

(72) Inventor: **Naipawer, Richard E., 9 Iris Lane, Wallington N.J. 07055(US)**

(74) Representative: **Urech, Peter, Dr. et al, Grenzacherstrasse 124 Postfach 3255, CH-4002 Basel(CH)**

**EP 0 203 528 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to (E)-3-methyl-5-(2,2,3--trimethylcyclopent-3-en-1-yl)pent-4-en-2-ol, more particularly the inventioned relates to the two diastereomers depicted by the formula

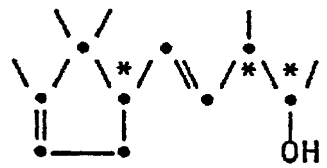

I

As it will be shown below, the formula Ia designates the diastereomers which possess the antio or 2R*, 3S* relative configuration at carbons 2 and 3, i.e. Ia stands for the (2R*, 3S*)-(E)-3-methyl-5-(2,2,3-trimethylcyclopent-3-en--l-yl)pent-4-en-2-ol. Ia and substantially pure Ia respectively, are odorants possessing a very intense creamy, woody, musk, sandalwood odour and are valuable ingredients for use in fragrance compositions for its musk and sandalwood-like qualities. The mixture of its diastereomers is more intense than any of the campholenic aldehyde derivatives that have been previously reported.

East Indian sandalwood oil has been described as being "perhaps one of the most precious perfumery materials from antiquity down to modern times, and its popularity has shown no signs of waning" [E. Guenther, "The Essential Oils", Vol. V, Page 173, D. Van Nostrand Company, Incorporated, New York (1952)]. This oil is widely used in perfumery and would be even more widely used except for its limited supply and high cost.

For many years a need existed for synthetic substitutes which could be used as sandalwood substitutes or as extenders. In 1977 Naipawer and Easter (US-PS 4,052,341) disclosed the compound 3-methyl-5-(2,2,3-trimethylcyclopent-3-en-1-yl)pentan-2-ol which was characterized by a tenacious fine soft woody odor similar to sandalwood oil and the naturally occurring santalols. This compound not only has a fine sandalwood odor, it is also more stable than the potentially labile allylic alcohols of beta- and alpha-santalol. [Naipawer and Easter also disclosed and claimed the unsaturated analog 3-methyl-5-(2,2,3-trimethylcyclopent-3-en-1-yl)pent-3-en-2-ol]. A major advantage of this invention is the fact that the compound can be readily and economically prepared from inexpensive materials, i.e., campholenic aldehyde and 2-butanone (methyl ethyl ketone). Campholenic aldehyde, which is readily available from alpha-pinene epoxide, is a relatively inexpensive starting material.

After the Naipawer and Easter patent appeared, there was a flurry of activity in this area and a number of patents appeared claiming sandalwood-type odorants starting from campholenic aldehyde. In most instances, these patents differed from one another in the compound that was condensed with campholenic aldehyde, the manner in which the condensation was conducted or the chemical steps that were performed on the condensation product.

The activity in the analysis of sandalwood oil and the search for synthetic substitutes has been thoroughly reviewed (See E.-J. Brunke and W. Rojahn, Dragoco Report (Eng. Frag. Ed.), No. 5, 67, (1980); E.-J. Brunke, ibid., No. 8, 187, (1981), E.-J. Brunke and E. Klein in "Fragrance Chemistry - The Science of the Sense of Smell", E.T. Theimer, Ed., Academic Press, New York, N.Y., 1982, pp 397-431; K.H. Shankaranarayana and K. Parthasarathi, Perfumer and Flavorist, 9, 17, (1984); and other references mentioned in those reviews.).

Based on a reading of the above review articles and the patents issued, it would appear that the prior art has exhaustively covered all of the more valuable odorants that could be made starting from campholenic aldehyde. Applicants, however, have found and disclose herein the most intense and most valuable campholenic aldehyde derivative that has been reported to date, a derivative that has escaped characterization and appreciation by all the investigators who have exhaustively studied and reviewed this field.

The present invention is more clearly understood by considering the sequence of steps outlined in Chart 1 which represents the most economical route for the preparation of what appears to be the most powerful odorant of this type ever prepared, i.e. the compound of formula I. [The asterisk (*) indicates an asymmetric center.]

## Chart 1

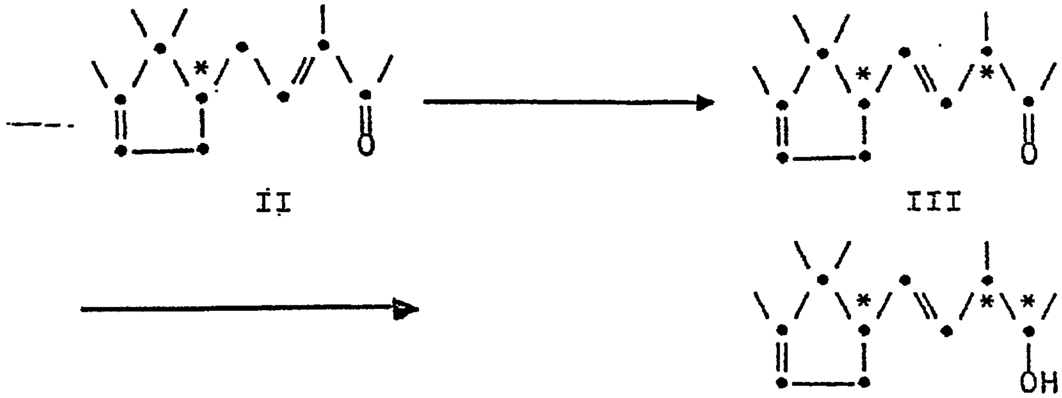

II

III

I

Compound II is an α,β-unsaturated ketone which can be prepared as described in US-PS 4,052,341. It can be isomeric about the double bond (E or Z) and has one asymmetric center which is designated by the asterisk (*). This compound II, can be converted to the β,γ-unsaturated ketone III by forming the enolate and protonating under conditions that give the kinetically favored product.

The configuration about the double bond in compound III is trans or E as indicated. The conversion of II to III introduces a second asymmetric center, also indicated by an asterisk (*). There are expected two possible diastereomers, i.e. two d,l pairs, for the ketone III. While the diastereomers have not been separated on gas-liquid chromatography (GLC) (on a polyethyleneglycol stationary phase, e.g. on Carbowax$^R$ 20 M capillary column), the $^{13}$C-NMR provides evidence that both diastereomers are present.

Compound III can be converted to compound I by reducing the ketone carbonyl to the corresponding alcohol. This process introduces a third asymmetric center (*) which means that four diastereomers (four pairs of enantiomers or d,l pairs) are now expected. In this instance, gas-liquid chromatography (Carbowax$^R$ 20 M capillary column) separates the product into two peaks. These two components are also separated by spinning band distillation. The component which is lower boiling and which elutes first on a Carbowax$^R$ 20 M capillary GLC column is hereinafter referred to as component Ia. The component which is higher boiling and which elutes last on a Carbowax$^R$ 20 M capillary GLC column is hereinafter referred to as component Ib. While each of these two components appear to be pure on the Carbowax$^R$ 20 M capillary column, gas-liquid chromatographic analysis on an ethylene glycol succinate capillary column (LAC-4-R-886) resolves each of these into two peaks. Therefore all four possible diastereomers (d, l pairs) are present. $^{13}$C-NMR and $^1$H-NMR (400 MHz) confirm the presence of the four diastereomers.

It could be assumed that two of the diastereomers would possess the anti (or threo) relative configuration with respect to the adjacent asymmetric centers at carbons 2 and 3, while the other two diastereomers would possess the syn (or erythro) relative configuration with respect to these two asymmetric centers.

Based on an examination of the 400 MHz $^1$H-NMR spectra of components Ia and Ib, it has been concluded that the two diastereomers present in Ia are of similar relative configuration as to the two adjacent asymmetric centers. A similar conclusion has been made as to the 2 diastereomers present in Ib. This conclusion is based on the fact that the spectrum for Ia shows a series of double peaks for the methyl groups on the side-chain, i.e. these methyl groups have nearly identical chemical shifts. The spectrum for component Ib also shows a series of such doublets, but the side-chain methyl groups in Ib have chemical shifts in Ia. Based on the differences in the chemical shifts for the side-chain methyl groups it can be concluded that the side-chains in Ia have different relative configurations, from the side chains in Ib i.e., one is anti and one is syn.

X-ray diffraction crystallographic analysis of crystalline esters of component Ia and of component Ib were utilized to determine the relative configuration at the side-chains in each component and the relative stereochemical configuration of the four diastereomers.

The first eluting diastereomer of component Ia (GLC on LAC-4R-886) produced a crystalline allophanate derivative suitable for X-ray diffraction crystallographic analysis. The diffraction pattern showed that this diastereomer possessed the anti relative configuration with respect to carbons 2 and 3 since the relative stereochemical configurations at these carbon atoms were determined to be R* and S*, respectively. The pattern also showed that the relative stereochemical configuration at ring carbon 1 was S*. The first component of Ia was therefore the (2R*, 3S*)-(E)-3--methyl-5-(2,2,3-trimethylcyclopent-3-en-1(S*)-yl)pent-4-en-2-ol diastereomer (d,l pair). Based on the $^1$H-NMR data (discussed above) indicating that both diastereomers present in Ia were of similar relative configuration at the side-chain, the second diastereomer of component Ia also possessed the anti relative configuration with respect to carbons 2 and 3 and was therefore the (2R*, 3S*)-(E)-3--methyl-5-(2,2,3-trimethylcyclopent-3-en-1(R*)-yl)pent-4-en-2-ol diastereomer (d,l pair).

The first eluting diastereomer of component Ib (GLC on LAC-4R-886) produced a crystalline carbamate derivative suitable for X-ray diffraction crystallographic analysis. The diffraction pattern showed that this pair possessed the relative stereochemical configuration at carbons 2 and 3 of R* and R* respectively. This diastereomer would therefore have the syn relative configuration with respect to these two carbon atoms. The diffraction pattern also showed that the relative stereochemical configuration at ring carbon 1 was R*. The first component of Ib was therefore the (2R*, 3R*)-(E)-3-methyl-5-(2,2,3-trimethylcyclopent-3-en-1(R*)-yl)-pent-4-en-2-ol diastereomer (d,l pair). Based on the $^1$H-NMR data discussed above) indicating that both diastereomers present in Ib were of similar relative configuration at the side-chain, it was concluded that the second diastereomer of component Ib also possessed the syn relative configuration at carbons 2 and 3 was therefore the (2R*, 3R*)-(E)-3- -methyl-5-(2,2,3-trimethylcyclopent-3-en-1(S*)-yl)pent-4-en-2--ol diastereomer (d,l pair).

Therefore, Ia designates the diastereomers of compound I which are lower boiling, elute first on a Carbowax[R] 20 M capillary column, and which possess the anti or R*, S* relative configuration at carbons 2 and 3, i.e. the (2R*, 3S*)-(E)-3-methyl-5-(2,2,3-trimethylcyclopent-3-en-1-yl)pent--4-en-2-ol isomers. Similarly Ib designates the diastereomers of compound I which are higher boiling, elute last on a Carbowax[R] 20 M capillary column, and which possess the syn or R*, R* relative configuration at carbons 2 and 3, i.e. the (2R*, 3R*)-(E)-3-methyl-5-(2,2,3-trimethylcyclopent-3-en-1-yl)pent-4-en-2-ol isomers.

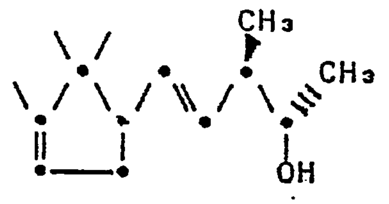

"anti"diastereomer (Ia)

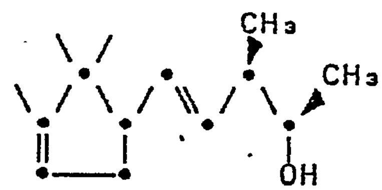

"syn" diastereomer (Ib)

The component designated as Ia is the campholenic aldehyde derivative having the strongest odor ever reported. It has about four hundred times the odor intensity, as determined by odor threshold values, of natural santalol and has about five hundred times the odor intensity of component Ib. (The odor threshold is a physical property of the composition and is defined as being "its lowest concentration in air that can be consistently distinguished from pure air" [J.E. Amoore, "Fragrance Chemistry - The Science of the Sense of Semll", E.T. Theimer, Ed., Academic Press, New York, N.Y., 1982, pp 34-41]. The odor threshold value is expressed in terms of the weight of odorant per unit volumn of air as determined by olfactometry, a technique known to those skilled in the art and discussed by Amoore. In essence, odor threshold value is the smallest amount of odorant that must be present in a unit volume of air to be detected, i.e., distinguished from the air itself).

This invention illustrates the importance that the stereochemical configuration plays in the odor properties of these alcohols. At the heart of this invention is the fact that the relative configuration around the asymmetric centers in the homoallylic alcohol (E)-3-methyl-5-(2,2,3--trimethylcyclopent-3-en-1-yl)pent-4-en-2-ol appears to be critical with regard to odor intensity. As reported above, one component (Ia) appears to have an odor intensity as shown by threshold values of over five hundred times the odor intensity of the other component (Ib).

The present invention is thus drawn to the compound (2R*, 3S*)-(E)-3-methyl-5-(2,2,3-trimethylcyclopent-3-en-1-yl)pent-4-en-2-ol Ia and to the substantially pure component Ia respectively which have been assigned structures as indicated above, to novel compositions containing the novel compounds, and the use of these novel compounds in odorant compositions. Finally, the present invention is drawn to the process for the preparation of I ex III.

The term "substantially pure" designates in the present context a material of, preferably, at least 90% purity.

US-PS 4,173 585 (IFF) discloses mixtures of compounds that contain as minor constituents, certain compounds that are disclosed in the compositions claimed in this application.

Thus US-PS 4,173,585 contains a sequence at columns 25 and 26 that shows that an aldol condensation produces a mixture of α,β-unsaturated methyl ketones, α,β-unsaturated ethyl ketones, β,γ-unsaturated methyl ketones, and β,γ-unsaturated ethyl ketones. Eight possible isomers are indicated but only four major peaks are indicated in Figure 4. There is no designation as to which peak is connected to which spectra or which compound, but it was possible to show that the smallest peak, peak A, contains the isomers of 3-methyl-5-(2,2,3-trimethylcyclopent-3-en-1-yl)pent-4-en-2-one, i.e. the β,γ methyl ketone of formula III. As US-PS 4,173,585 shows, peak A contains both the E and Z isomers of the β,γ methyl ketone. Thus the E isomers in admixture with the Z isomers of 3-methyl-5-(2,2,3-trimethyl-

cyclopent-3-en-1-yl)pent-4-en-2-one is a minor constituent contained in the product prepared in US-PS 4,173,585 by condensing campholenic aldehyde and methyl ethyl ketone using a zinc acetate catalyst. (Based on Figure 4 of US-PS 4,173,585, peak A seems to account for about 5% to 8% of the total product, i.e. as encompassed by the four peaks.)

Reduction of this product, as carried out in Example IC of US-PS 4,173,585 could only produce small amounts of the E isomer, in admixture with the Z isomer, of 3-methyl-5--(2,2,3-trimethylcyclopent-3-en-1-yl)pent-4-en-2-ol. While at column 29 it is stated that preparative GLC yielded 6 peaks designated as A, B, C, D, E and F, there is no disclosure as to:

a) which peaks belonged to which structures, there being eight structures given for the six peaks mentioned,

b) whether any of the compounds were observed in reasonably pure form, or whether the samples observed from the gas-liquid chromatograph (see figure 12) were mixtures,

c) which peaks had which odor properties, or

d) the relative abundance of each compound in the crude mixture.

No reference in the prior art teaches the isolation, characterization and odor properties of the (2R*, 3S*)-(E)--3-methyl-5-(2,2,3-trimethylcyclopent-3-en-1-yl)pent-4-en--2-ol, of the (2R*, 3R*)-(E)-3-methyl-5-(2,2,3-trimethylcyclopent-3-en-1-yl)pent-4-en-2-ol, or of their mixture, the (E)-3-methyl-5-(2,2,3-trimethylcyclopent-3-en-1-yl)-pent-4-en-2-ol. There is also no teaching of the preparation of the intermediate (E)-3-methyl-5-(2,2,3-trimethylcyclopent-3-en-1-yl)- pent-4-en-2-one in a form suitable for preparing the novel compounds and compositions of the present invention.

As indicated in Chart 1, the mixture of isomers of (E)-3-methyl-5-(2,2,3-trimethylcyclopent-3-en-1-yl)pent-4-en-2-ol (I) can be prepared by converting the α,β-unsaturated ketone II to the β,γ-unsaturated ketone III - i.e. it can be isomerized to III - and subsequently converting the carbonyl group of III to an alcohol. The compound II can be prepared in a variety of ways, two of which are illustrated in Chart 2 below [The asterisk (*) is used in Chart 2 and throughout this text to indicate a pertinent asymmetric center.]

The scope and limitations of this invention as well as the preferred embodiments of this invention are best understood by following the scheme as outlined in Chart 2 since the novel compositions reported herein which contain Ia may contain other isomers which result from the particular process used.

## Chart 2

Path A

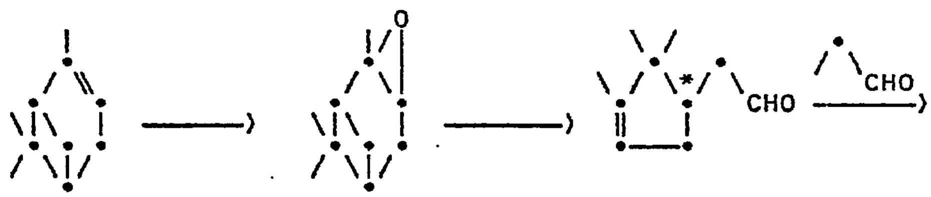

IV V VI

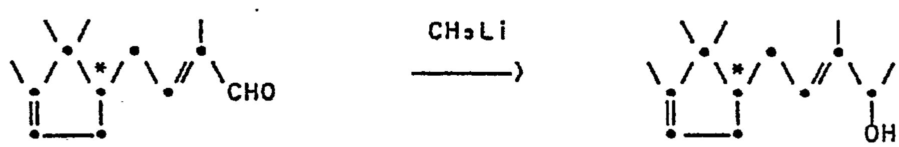


X XI

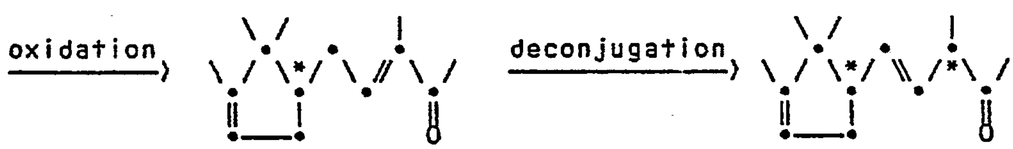


II III

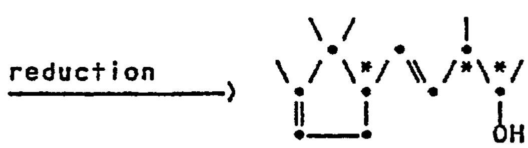


I

Path B

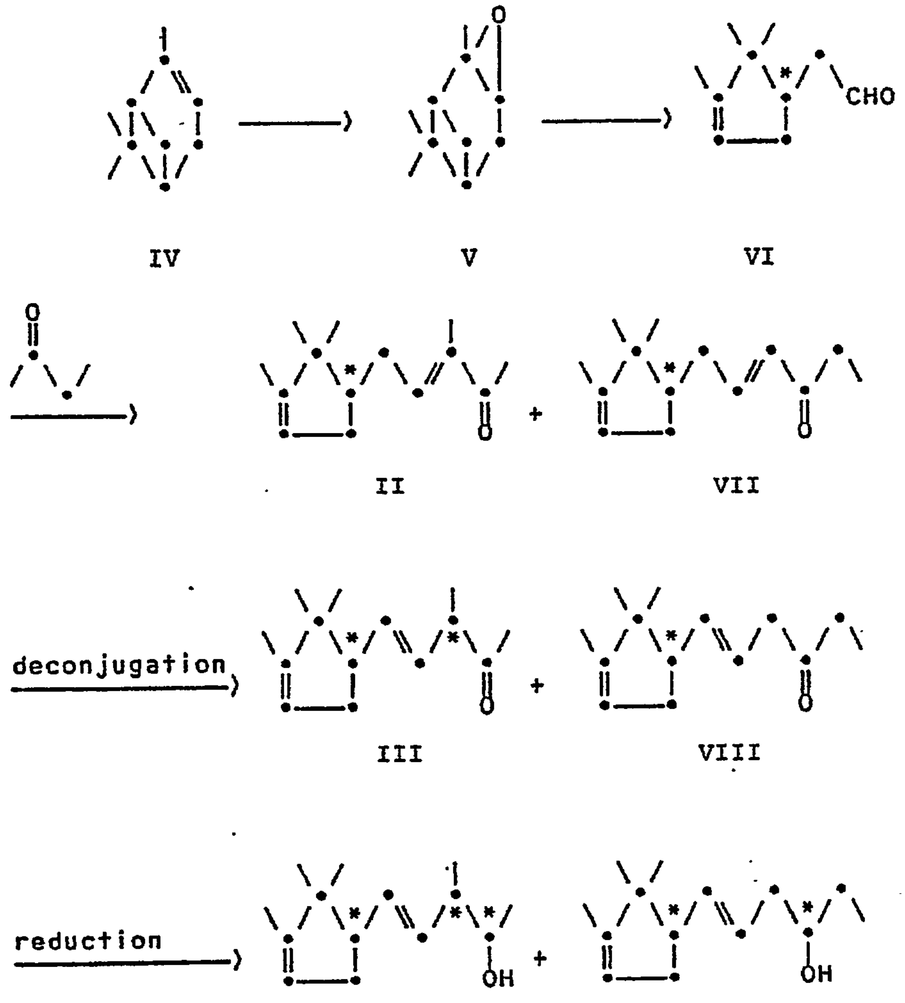


IV V VI

II VII

III VIII

I IX

Chart 2 shows two possible routes to compositions containing compound I, i.e. the mixture of diastereomers of (E)-3-methyl-5-(2,2,3-trimethylcyclopent-3-en-1-yl)pent-4-en-2-ol. Campholenic aldehyde (VI) is the common starting material for both Path A and Path B and is prepared from α-pinene (IV) via the epoxide V, as shown in Chart 2. This is a process well known in the art, see for example US-PS 4,052,341, Example 1.

Path A differs from Path B in that a different compound is used in the aldol condensation with the campholenic aldehyde. The former uses propanal and the latter uses 2-butanone. Path A has the advantage that the condensation product X can be converted to the α,β-unsaturated ketone II without forming, at the same time, small amounts of the ethyl ketone VII. Path B, however, is much more economical to carry out than Path A since Path B requires fewer and less expensive steps. These economic advantages make Path B the preferred route even though some of the ethyl ketone, VII, would be formed during the condensation of campholenic aldehyde and 2-butanone. (The ethyl ketone VII is eventually converted to the alcohol IX which, as will be shown below, has little effect on the odor quality of the final product.)

In Path A, the condensation of campholenic aldehyde and propanal can be carried out as described in Example IV-1 of US-PS 4,052,341 to form the desired 2-methyl-4-(2,2,3-trimethylcyclopent-3-en-1-yl)but-2-enal (X). The aldehyde X can then be converted to the alcohol XI using methylmagnesium halide or methyllithium in a Grignard-type reaction. The alcohol XI can then be oxidized to the pure ketone II which is free from any of the ethyl ketone isomer VII. The ketone II can be deconjugated to III which in turn can be reduced to the alcohol I as described below in the discussion of the preferred route, Path B.

In Path B the aldol condensation between campholenic aldehyde and 2-butanone may take place at either of the carbon atoms adjacent to the carbonyl and may produce a mixture of products. It is expected that an aldehyde will preferentially react with the more reactive methylene group than with the less reactive methyl. The relative amounts of methyl ketone or ethyl ketone formed may be dependent on variables such as the reaction temperature, reaction time and catalyst used.

Using the conditions described in US-PS 4,052,341, the ratio of compound II to compound VII is about ten to one. It has been found that if the ketone VII is carried through the sequence of steps, the compound formed, i.e. the 6-(2,2,3--trimethylcyclopent-3-en-1-yl)hex-5-en-3-ol (IX), has an odor which is much weaker than and compatible with the odor of Ia, and therefore there is no need to separate the ten to one mixture of II and VII before proceeding.

In the aldol condensation between campholenic aldehyde and 2-butanone, shown in Path B, α,β-hydroxyketone intermediate is formed which is capable of dehydrating to produce either the α,β-unsaturated ketone II or the β, γ-unsaturated ketone III. The α,β-unsaturated ketone II is usually the major or exclusive product. (The amount of the β,γ-unsaturated ketone that may be produced is usually dependent on variables such as reaction temperature, reaction time, catalyst and substituent groups present on the aldehyde and/or the ketone being condensed. See H.O. House, "Modern Synthetic Reactions", 2nd ed., W.A. Benjamin, Inc., Menlo Park, CA., 1972, pp 644 - 645.)

Once the ketone II has been obtained via Path A or Path B, it can be converted to the β,γ-unsaturated ketone III by any means known in the art for moving double bonds out of conjugation [H.J. Ringold and S.K. Malhotra, Tet. Letters, No. 15, 669 (1962); E. D'Incan and P. Viout, Tetrahedron 40, 3421 (1984)]. All these methods rely on the initial formation of an enolate followed by the protonation of that enolate under conditions that provide the kinetically favored product in excess over the thermodynamically favored product.

While most of the conditions for converting an α,β-unsaturated ketone to the corresponding β,γ-unsaturated ketone should be suitable, it is preferred to use conditions that minimize undesirable side reactions such as self-condensation of the ketones and isomerization of the kinetically favored β,γ-unsaturated product back into the thermodynamically favored α,β-unsaturated starting material.

In the preferred process, the ketone to be isomerized is slowly added to a solution of a suitable base (e.g. an alkali metal tertiary alkoxide) in an appropriate aprotic solvent at a temperature of about 10°C to 30°C. The rate of addition would be determined by one skilled in the art depending on the size of the reaction, the productivity required and the amount of self-condensation that can be tolerated. For laboratory-size batches, an addition time of one hour is suitable.

The temperature of the enolate formation is not critical. Temperatures abov 30°C tend to produce more condensation side products and temperatures below 10°C do not offer a sufficient advantage to justify the need for the additional cooling. Once the addition is complete, the reaction is continued at 10°C to 30°C for an additional time until the enolate formation is complete. (Completion of enolate formation can be determined by the workup of a small sample and analysis of the products by gas-liquid chromatography.)

As the base used for the isomerization, it is preferred to use an alkali metal tertiary alkoxide such as potassium t-butoxide, sodium t-butoxide, the corresponding t-amylates or the like. The readily available and more economical potassium t-butoxide is preferred. It is preferred to use an excess of the base (20-80%) with an excess of about 40% to 60% being especially preferred.

The solvent used is limited to aprotic, anhydrous solvents which are capable of solvating the t-alkoxides and the enolate formed. Typical solvents normally used for this purpose are ethers, polyethers, N,N-dialkylamides and the like. Preferred solvents are the commercially available 1,2-dimethoxyethane (DME), diethylene glycol dimethyl ether (Diglyme), N,N-dimethylacetamide (DMAC) and N,N-dimethylformamide (DMF), with DMF being especially preferred.

Once the enolate has been formed, it can be protonated to the β,γ-unsaturated ketone by means of a suitable proton source which will allow the kinetic products (the β,γ-unsaturated ketone) to be formed without reverting to the α,β-unsaturated ketone. While a number of proton sources should be suitable, it has been found that excellent results can be obtained by either adding aqueous acetic acid to the reaction mixture or by adding the reaction mixture to aqueous acetic acid. The β,γ-unsaturated ketone is obtained in 75-85% yields and contains less than 5% of the starting α,β-unsaturated ketone. (Since the neutralization is exothermic, it is preferred to use cold aqueous acetic acid). The desired β,γ-unsaturated ketone can be separated from the small amount of the higher boiling α,β-unsaturated ketone present by fractional distillation. (The presence of small amounts of the α,β-unsaturated ketone II 3-methyl-5-(2,2,3-trimethylcyclopent-3-en-1-yl)pentan-2-ol (XII) and 3-methyl-5-(2,2,3-trimethylcyclopent-3-en-1-yl)-pent-3-en-2-ol (XI), both of which have odors that are compatible with the desired product.

If the α,β-unsaturated ketone II was prepared by Path A, the β,γ-unsaturated ketone III is free of any of the compound VIII, and can be obtained in extremely pure form. If the α,β-unsaturated ketone II was prepared by Path B, and contained the ethyl ketone VII, the ethyl ketone present will be isomerized to the β,γ-unsaturated ketone VIII. It has been found, however, that the enolate of the β,γ-unsaturated ketone VIII forms self-condensation products much more rapidly than does the β,γ-unsaturated ketone III under basic conditions. It has also been found, that if the isomerization is carried out a little longer or at somewhat higher temperatures, ca. 40° - 60°C, the β,γ-unsaturated ketone VIII present will consume itself via self-condensation. By following the reaction by GLC, the reaction can be run until the oly β,γ-unsaturated ketone present is compound III and virtually none of the compound VIII is present. Thus one can prepare pure III even though Path B is used.

The β,γ-unsaturated ketone obtained via either Path A or Path B can be converted to the corresponding homoallylic alcohol by reducing the carbonyl group to a hydroxyl group. The reduction can be suitably carried out using a metal hydride such as lithium aluminium hydride, sodium borohydride, sodium bis(2-methoxyethoxy)aluminium hydride, etc., with sodium borohydride being preferred because of economic considerations and ease of handling.

The product resulting from the reduction, as mentioned earlier, is primarily a mixture of diastereomers having the structure I. The three asymmetric centers of compound I allow for the existence of four diastereomers, i.e., four d,l pairs.

Gas-liquid chromatographic analysis (Carbowax$^R$ 20 M, fused silica capillary) shows only two peaks present in approxi- mately a 7 to 5 ratio, each peak of which can be isolated pure by a careful distillation, e.g. a spinning band distil- lation. These two components have been designated as Ia and Ib, respectively. GLC analysis on an ethylene glycol succi- nate (LAC-4-R-886) capillary column shows that Ia and Ib are each composed of two peaks (each in an approximate 1 to 1 ratio) indicating that all four possible diastereomers are present. [$^{13}$C-NMR and $^{1}$H-NMR (400 MHz) also confirm the presence of all four possible diastereomers.].

As mentioned earlier, it has been determined from X-ray diffraction crystallographic analysis and $^{1}$H-NMR (400 MHz) that the two diastereomers denoted by Ia possess the anti or R*, S* relative configuration at the two asymmetric centers in the side-chain (carbons 2 and 3), while the two diastereomeric pairs denoted by Ib possess the syn or R*, R* relative configuration at these carbons.

Alcohol Ia therefore denotes the more abundant component which has the lower boiling point, elutes first on a Carbowax$^R$20 M capillary GLC column, and contains the two diastereomers assigned the anti or R*, S* stereochemical configuration in the side-chain, i.e. the (2R*, 3S*)-(E)-3-methyl-5-(2,2,3-trimethylcyclopent-3-en-1-yl)pent-4-en-2-ols. Alcohol Ib denotes the less abundant component which has the higher boiling point, elutes second on a Carbowax$^R$ 20 M capillary GLC column, and contains the two diastereomers assigned the syn or R*, R* stereochemical configuration in the side-chain, i.e. the (2R*, 3R*)-(E)-3-methyl-5-(2,2,3--trimethylcyclopent-3-en-1-yl)pent-4-en-2-ols.

In evaluating the odor properties of the alcohol I it was observed that component Ia had an odor of unexpected intensity, particularly in comparison to related sandalwood-like odorants known in the prior art and also in comparison to component Ib. These differences were shown by subjecting a number of odorants to an olfactometric determination of their respective odor threshold values.

The relative odor threshold values of alcohols Ia and Ib, and related odorants known in the prior art were determined by standard olfactometric methods known to one skilled in the art. These values are tabulated in Table I. The values are relative to the saturated compound XII which was assigned a value of 1.0.

Table I

| Odorant | Relative Odor Threshold |
| --- | --- |
| 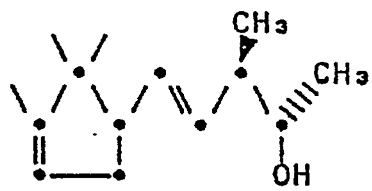 Ia | 0,003 |
| 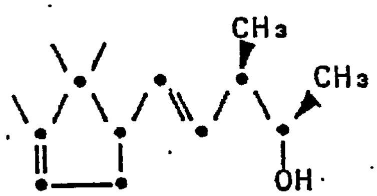 Ib | 1,5 |

## Table I (continuation)

| Odorant | Relative Odor Threshold |
|---|---|
| α-Santalol, S. Arctander, Perfume and Flavor Chemicals, Montclair, N.J. USA, 2, (1971), item 2819 | 1,17 |
| 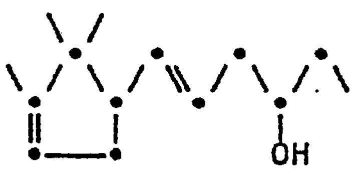<br>IX | 7,83 |
| 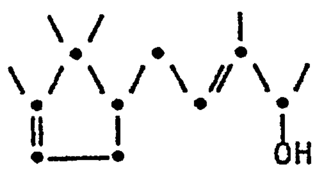<br>XI<br>US-PS 4,052,341 | 0,13 |
| 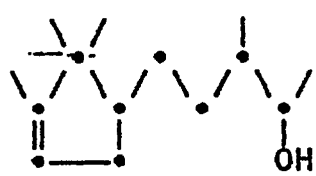<br>XII<br>US-PS 4,052,341 | 1,0 |

The above relative odor threshold values show the unexpected and unpredictable superiority of Ia. The odor of the alcohol Ia is approximately 300 times more intense than the odor of XII, which, as discussed previously, is an intense sandalwood odorant of good stability. The odor of component Ia is approximately 400 times more intense than the odor of α-santalol and approximately 40 times more intense than the odor of the corresponding α,β-unsaturated alcohol XI. Even more surprising and unexpected is the fact that the odor of Ia is more than 500 times as intense as the odor of its sister diastereomeric alcohol Ib.

While pure Ia would be the strongest sandalwood-type odorant ever discovered, it is not economical to separate this component from Ib. Fortunately Ib is a much weaker odorant, 500 times weaker with respect to odor threshold value, and does not interfere with the odor properties of Ia. In the mixture of Ia and Ib, the Ib is essentially a diluent.

It is because of the extreme odor intensity of Ia, it being several orders of magnitude more powerful than any of the other isomers shown in Table I, that mixtures containing Ib, IX, XI or XII need not be separated. For example, since it takes five hundred times more of Ib to be detected in air than Ia, a mixture which is about 60% Ia and about 40% Ib is going to have an odor which is very similar to pure Ia. the same is true for any amount of isomers due to the use of Path B, e.g. compound IX which may have resulted from the presence of ketone VII due to aldol condensation on the methyl group of 2-butanone. Since Table I shows that there must be over a thousand times more compound IX than Ia to be detectable in air, then even if compound IX is present in about ten percent, there will be no detrimental effect on the odor of the mixture and that odor will be very similar to the odor of pure Ia.

Even if there was some α,β-unsaturated ketone II present with the β,γ-unsaturated ketone III after the isomerization step, this would be converted to the saturated pentanol XII, and the allylic alcohol XI. Both of these compounds are fine and strong odorants, but small amounts would not discernably detract from the odor value of Ia.

The novel compositions of this invention comprise, therefore, preferably any compositions wherein the content of Ia is greater than ten percent of the total composition. The preferred compositions of this invention are those composi- tions wherein Ia is greater than twenty-five percent of the total, with those compositions having greater than forty percent Ia being highly preferred. Especially preferred are those compositions wherein the amount of Ia present is greater than fifty percent.

Among the especially preferred compositions are those consisting essentially of

a) fifty to one hundred percent Ia
b) zero to forty-five percent Ib
c) zero to twelve percent compound IX
d) zero to ten percent compound XI
e) zero to ten percent compound XII.

As mentioned earlier, it is not economically practical to separate Ia and Ib so that the commercially successful product will be a mixture of these two components. Said mixtures will consist essentially of

a) forty to sixty-five percent Ia
b) twenty-five to forty-five percent Ib
c) zero to twelve percent compound IX
d) zero to five percent compound XI
e) zero to five percent compound XII.

The reason that these novel components are so valuable in perfumery is due to their very intense and unique odor properties. Alcohol Ia is characterized not only as sandalwood-like, but as having qualities that make it even more valuable. Its unique odor can be characterized as a powerful, creamy, woody, musk, sandalwood odor. The unique intensity along with the musk quality gives Ia a value possessed by no other substance or combination of substances and makes it a uniquely valuable odorant in a variety of fragrance types such as woody compositions, musk-like compositions, floral compositions, chypre accords, citrus accords and the like. Alcohol Ia lends to these fragrances the natural quality usually found only when the more expensive natural oils are used, yet it is unique in its own right and cannot be replaced by any natural oil or combination thereof. Since the intensity of Ia is so strong, the composition consisting of Ia plus Ib or compositions of Ia plus Ib which also include some amounts of IX and/or XII and/or XI can be used with practically the same effect as pure Ia. The odor of Ia dominates these compositions. The other isomers (Ib, IX, XI, XII) affect the mixture only by reducing its intensity, in effect, acting as diluents.

The addition of the novel (compounds or) compositions of this invention containing Ia to fragrance compositions results in dramatic improvements in a number of fragrance types. A number of illustrations showing the value of the compositions of this invention are given in Example 6. In this example, compositions of this invention were added such that the amount of Ia present in the final fragrance mixture was from about 0,15% to 2,5%. The effects were dramatic and could not be achieved by substituting similar amounts of sandalwood oil or any of the other odorants shown in Table I.

In fragrance compositions of the woody type (precious woody or cedar-like) one can use the novel (compounds or) compositions of this invention containing Ia in larger amounts to add a distinctive and dominant note, or in smaller amounts to provide a roundling or blending effect. The compositions of this invention can be used similarly in large amounts in musk-like compositions to provide unique and highly desirable characteristics.

In floral type compositions, the use of small amounts of the novel compositions of this invention is found to contribute in a way that results in a rounder, warmer and better blended fragrance. In citrus accords, the addition of small amounts of the compositions of this invention adds an interesting musky note to these accords. The compositions of this invention have also been shown to enhance the woody notes of a chypre accord.

The novel (compounds or) compositions of this invention containing Ia can be used in a variety of applications. The amounts used and the scope of such use will depend solely on the imagination and personal preferences of each perfumer.

For the most part, the novel (compounds or) compositions of this invention can be used in fragrance formulations in an amount such that the amount of Ia present in the final fragrance mixture can range from as low as ca. 0,005% to ca. 5% with ca. 0,15% to ca. 2,5% being preferred. This will vary, of course, depending upon the type of fragrance formula involved. Concentrations above ca. 5%, even as high as 80 or 90%, may be used successfully for special effects.

The novel (compounds or) compositions of this invention can be used to prepare fragrance bases for the preparation of, e.g. perfumes and toilet waters according to methods known to the perfumer, e.g. by adding the usual diluents, e.g. alcoholic and aqueous diluents thereto. Approximately 15-20% by weight of base would be used for perfumes and approximately 3-5% for toilet waters.

Similarly, the base compositions can be used to odorize soaps, detergents, cosmetics or the like. In these instances, a base concentration of from about 0,1% to about 2% by weight can be used.

It is a matter of course, that, instead of using the novel compositions mentioned above, (substantially pure) 2R*, 3S*-(E)-3-methyl-5-(2,2,3-trimethylcyclopent-3-en-1--yl)pent-4-en-2-ol can be used instead.

The following examples are provided to illustrate the preferred embodiments as they are disclosed herein and are not to be construed as limiting.

Infrared spectra (IR) were recorded as neat samples using a Perkin-Elmer Model 681 spectrophotometer and absorptions are reported in inverse centimeters ($cm^{-1}$).

Molecular weights were determined by electron impact mass spectroscopy using a Finnigan Model 4000 quadrapole mass spectrometer.

Nuclear magnetic resonance (NMR) spectra were recorded as solutions in chloroform-$d_1$, using a Varian EM-360 proton spectrometer ($^1H$-NMR) operating at 60 MHz, a Brüker Model WH-400 heteronuclear spectrometer ($^1H$-NMR) operating at 400 MHz and a Varian Model CFT-20 heteronuclear spectrometer ($^{13}C$-NMR), and are reported as δ units relative to tetramethylsilane (TMS) (0,0δ). Unless otherwise indicated, $^1H$-NMR spectra were recorded on the Varian Model EM-360 spectrometer operating at 60 MHz.

Unless otherwise indicated gas-liquid chromatography (GLC) was carried out on a Carbowax$^R$ 20 M (registered trademark of Union Carbide Corp. for polyethylene glycol) fused silica capillary column (0,25 mm × 30 meters) using a Hewlett-Packard Model 5880A gas chromatograph with a flame ionization detector (FID). Non-routine GLC, where indicated, was carried out on an LAC-4-R-886 (ethylene glycol succinate) capillary column (0,30 mm × 39 meters).

Unless otherwise indicated weights are in grams, temperatures are in degrees centigrade (°C), pressures are in mmHg and yields are based on theory.

## Example 1

Preparation of (E)-3-methyl-5-(2,2,3-trimethylcyclopent-3-en-1-yl)pent-4-en-2-one (III)

The α,β-unsaturated ketone used as starting material in this example was obtained according to Example II-1 of US-PS 4,052,341 and consists primarily of 3-methyl-5-(2,2,3--trimethylcyclopent-3-en-1-yl)pent-3-en-2-one (II) (82%) and 6-(2,2,3-trimethylcyclopent-3-en-1-yl)hex-4-en-3-one (VII) (6-8%) and small amounts of the corresponding β,γ isomers III, (4%) and VIII (4%).

Potassium t-butoxide (33,6 g, 0,30 mole) was added to a reaction flask containing 150 ml of N,N-dimethylformamide (DMF). The mixture was stirred and was cooled to 15°C. The α,β-unsaturated ketone (41,2 g, 0,2 mole) was added slowly over 1,0 hour at 15-25°C, with cooling being applied as necessary in order to maintain the temperature within this range. The resultant solution was stirred at ambient temperature for an additional period of 4,0 hours.

The mixture was cooled to 0°C and 150 ml of aqueous 20% acetic acid was added rapidly. The mixture was stirred for 15 minutes, was then poured into a beaker containing 150 ml of water and was extracted with three 100-ml portions of toluene. The toluene extracts were combined and were washed with two 100-ml portions of 10% sodium bicarbonate solution and then with one 100-ml portion of water.

The washed toluene layer was dried over anhydrous magnesium sulfate, filtered and the toluene removed by distillation at reduced pressure (ca. 100 mmHg). The oil remaining was distilled at reduced pressure (0,5 mmHg) to yield 34,8 g (84,5 % yield) of (E)-3-methyl-5-(2,2,3-trimethylcyclopent-3-en-1-yl)pent-4-en-2-one (III) (86%) and the isomeric ethyl ketone VIII (9%): bp 73-86°C (0,5mmHg); mol. wt. 206 (MS); IR 1725 $cm^{-1}$ (carbonyl), 805 $cm^{-1}$ trisubstituted olefin).

$^1H$-NMR 0,8δ (3H,s), 1,0 (3H,s), 1,2 (3H, d, J ~ 7.5Hz), 1,6 (3H, broad s, olefinic $CH_3$), 2,2 (3H, s, acetyl $CH_3$), 1,9 - 2,5 (3H, broad complex), 3,2 (1H, broad multiplet, J ~ 7,5), 5,2 (1H, broad multiplet, olefinic H), 5,4-5,9 (2H, complex).

$^{13}C$-NMR 121,5 ppm (d) and 147,8 (s) representing 2 ring olefinic carbons; 129,9 (d) and 134,2 + 134,5 (2d) representing 2 olefinic carbons; 209,3 (s, carbonyl). The $^{13}C$-NMR indicates 14 distinct carbon resonances with 7 carbon resonances appearing as two resonances indicating the presence of two diastereomers of the (E)-3-methyl-5-(2,2,3--trimethylcyclopent-3-en-1-yl)pent-4-en-2-one.

(The procedure described above is essentially that outlined in Chart 2, Path B. Ketone III prepared via Path A would be free of the isometric ethyl ketone VIII. Also, if the starting ketone II was prepared from optically pure R- or S-campholenic aldehyde, as shown in US-PS 4,052,341, the asymmetric center that is part of the cyclopentyl ring would be fixed as R or S.)

Example 2

Preparation of (E)-3-methyl-5-(2,2,3-trimethylcyclopent-3-en-1-yl)pent-4-en-2-ol (I)

Ethanol (221g , 280 ml), water (70g) and 30% sodium hydroxide solution (1,0 ml) were added to a reaction flask. The mixture was stirred and sodium borohydride powder (12,9g, 0,34 mole) was added in portions. The mixture was cooled to 0°C and 116,2g (0,56 mole) of a composition prepared as described in Example 1 containing (E)-3-methyl--5-(2,2,3-trimethylcyclopent-3-en-1-yl)pent-4-en-2-one (III) (87%) and the isomeric ethyl ketone VIII (8%) was added slowly over 1,0 hour at 0-5°C.

The resultant mixture was stirred for 1,0 hour at 0°C and then for 3,0 hours at ambient temperature. The mixture was poured into a beaker containing 400 ml of water and 40 ml of 30% sodium hydroxide solution and stirred well. The aqueous mixture was then extracted with three 125-ml portions of hexane. The hexane extracts were combined, washed with three 150-ml portions of water, dried over anhydrous magnesium sulfate, filtered and concentrated by distilling off the hexane at atmospheric pressure (760mm).

The remaining oil was distilled at reduced pressure (0,9 mm) to give 103,9 g (94.5% yield) of (E)-3-methyl-5-(2,2,3--trimethylcyclopent-3-en-1-yl)pent-4-en-2-ol (I) and the ethyl isomer IX; GLC (Carbowax$^R$ 20 M capillary column) shows I to be two peaks present at 51% and 36%, respectively, and IX present at 7%; GLC (ethylene glycol succinate capillary column) shows I to be four peaks present at 27,5%, 27,0%, 19,5% and 19,5%, respectively, and IX present at 6,5%; bp 93-102°C (0,9mm); $n_D^{20}$ 1,481; mol. wt. 208. (MS); IR 3360 $cm^{-1}$ (hydroxy), 3035 (olefinic), 1650 (olefinic) and 790 (trisubstituted olefin); Odor: very intense, creamy, woody, musk, sandalwood.

$^1$H-NMR 0,8δ (3H, s), 1,0 (3H, s), 1,1 (3H, d, J ~ 7Hz), 1,2 (3H, d, J ~ 7 Hz), 1,6 (3H, broad s, olefinic $CH_3$), 1,9-2,6 (5H, broad complex), 3,6 (1H, broadened sextet), 5,2 (1H, broad multiplet, ring olefinic proton), 5,1-5,8 (2H, broad multiplet, 2 olefinic protons).

$^{13}$C-NMR 12,7 ppm (q), 16,0 + 16,7 (2q), 20,1 + 20,3 (2q), 20,5 (q), 25,4 + 25,5 (2q), 35,4(t), 44,1 + 44,4 (2d), 45,2 (d), 48,0 (s), 54,1 + 54,3 (2d), 70,1 + 71,0 (2d) 122,5 (d), 132,6 + 132,7 (2d), 133,7 (d) and 148,0 (s). The $^{13}$C-NMR indicates 14 distinct carbon resonances with 7 carbon resonances appearing as two resonances indicating the presence of diastereomers.

Example 3

A) Separation and purification of the two separable components of (E)-3-methyl-5-(2,2,3-trimethylcyclopent-3-en-1-yl)pent-4-en-2-ol (Ia and Ib) and the isomer (E)-6-(2,2,3-trimethylcyclopent-3-en-1-yl)hex-5-en-3-ol (IX)

The product prepared according to Example 2 was subjected to a spinning band distillation and the three components isolated in pure form and characterized by special analyses. The following results were obtained:

(1) (2R*, 3S*)-(E)-3-Methyl-5-(2,2,3-trimethylcyclopent-3--en-1-yl)pent-4-en-2-ol (Ia)

GLC (ethylene glycol succinate capillary column) two peaks present at 48,4% and 42,1 %, respectively; bp 86,5°C (0,95 mmHg); IR 3380 $cm^{-1}$ (hydroxyl), 3040 (olefinic), 1655 (olefinic), 800 (trisubstituted olefin); MS (m/e), 208 (M+, 1%), 164 (66%), 149 (84%), 121 (100%), 109 (50%), 108 (99%), 107 (97%), 93 (82%), 83 (50%), 55 (93%), 45 (84%), 43 (63%), 41 (80%); Odor Threshold Value 0,02 ng/l; Odor: powerful creamy, woody, musk, sandalwood.

$^1$H-NMR 0,8δ (3H,s), 1,0 (3H, s), 1,02 (3H, d, J ~ 6Hz). 1,2 (3H, d,J ~ 6,5 Hz), 1,6 (3H, broad s, olefinic $CH_3$), 1,9 - 2,6 (5H, broad complex), 3,6 (1H, heptet, J ~ 6,5Hz), 5,3 (1H, broad multiplet, ring olefinic proton), 5,1-5,9(2H, broad complex, 2 olefinic protons).

The $^1$H-NMR (400 MHz) spectrum is consistent with the structure of alcohol Ia and indicates a 1:1 mixture of diastereomeric alcohols.

$^{13}$C-NMR 12,7 ppm (q), 16,6 (q), 20,0 (q), 20,6 (q), 25,5 (q), 35,5 (t), 45,0 (t), 45,0 (d), 48,0 (s), 54,4 (d), 71,0 (d), 121,6 (d), 132,8 (d), 133,3 (d), 147,9 (s).

(2) (2R*, 3R*)-(E)-3-Methyl-5-(2,2,3-trimethylcyclopent-3--en-1-yl)pent-4-en-2-ol (Ib)

GLC (ethylene glycol succinate capillary column) two peaks present at 43,3% and 50,3%, respectively; bp 93,5°C (1,20 mmHg); IR 3350 3350 $cm^{-1}$ (hydroxyl), 3040 (olefinic), 1655 (olefinic) and 798 (trisubstituted olefin); MS(m/e), 208 (M+, absent), 164 (82%), 149 (86%), 121 (100%), 109 (48%), 108 (95%), 107 (96%), 93 (79%), 83 (51%), 55 (90%), 45 (81%), 43 (60%), 41 (75%); Odor Threshold Value 9,0 ng/l; Odor: Woody, sandalwood, with fruity, vetiver acetate nuances.

$^1$H-NMR 0,8δ (3H,s), 1,0 (3H,s), 1,1 (3H, d, J ~ 7Hz), 1,2 (3H, d, J ~ 7Hz), 1,6 (3H, broad s), 1,9-2,5 (4H, broad complex), 2,7 (1H, broad s, exchanged with $D_2O$), 3,6 (1H, multiplet, J ~ 6Hz), 5,3 (1H, broad multiplet, ring olefinic proton), 5,1-5,8 (2H, broad complex, 2 olefinic protons).

The $^1$H-NMR (400 MHz) spectrum is consistent with the structure of alcohol Ib and indicates a 1:1 mixture of diastereomeric alcohols.

$^{13}$C-NMR 12,6 ppm (q), 16,2 (q), 20,2 (q), 20,5 (q), 25,5 (q), 35,5 (t), 44,5 (d), 48,0 (s), 54,4 (d), 71,2 (d), 121,6 (d), 132,2 (d), 132,8 (d) and 147,9 (s).

(3) (E)-6-(2,2,3-Trimethylcyclopent-3-en-1-yl)hex-5-en-3-ol (IX)

bp 101,5°C (1,40 mmHg); IR 3345 cm$^{-1}$ (hydroxyl), 3040 (olefinic) and 800 (trisubstituted olefin); MS(m/e) 208 ($M^+$, 4%), 150 (45%), 135 (100%), 108 (48%), 107 (83%), 93 (48%), 59 (50%), 41 (36%); Odor Threshold Value 47 ng/l; Odor: Woody, ionone, with fruity, maple nuances.

$^1$H-NMR 0,8δ(3H, s), 0,95) (3H, t, J ~ 7Hz), 1,0 (3H, s), 1,1-1,7 (2H, multiplet, J ~ 6Hz), 1,6 (3H, broad s, olefinic $CH_3$), 1,9-2,7 (6H, broad complex), 3,6 (1H, broad multiplet, J ~ 6Hz), 5,3 (1H, broad multiplet, ring olefinic H), 5,4-5,9 (2H, broad complex, 2 olefinic protons).

$^1$H-NMR (400 MHz) spectrum is consistent with the structure of alcohol IX and indicates a 1:1 mixture of diastereomeric alcohols.

$^{13}$C-NMR 9,9 ppm (q), 12,7 (q), 20,5 (q), 25,5 (q), 29,4 (t), 35,5 (t), 40,5 (d), 47,9 (s), 54,3 (t), 72,4 (d), 121,5 (d), 126,9 (d), 134,8 (d), 147,9 (s).

B) Identification of the diastereomers of (E)-3-methyl-5--(2,2,3-trimethylcyclopent-3-en-1-yl)pent-4-en-2-ol by X-ray diffraction crystallography:

The product (70 g; 0,34 mole) prepared according to Example 2 was reacted with 60 g (0,36 mole) α-naphthyl isocyanate at 120° - 130°C. The resultant mixture was cooled to ambient tamperature and was dissolved in hexane (300 ml). The hexane solution was cooled to 0°C in order to induce crystallization. The crystalline material was collected by filtration to give 57 g of solid α-naphthyl carbamate (mp 90° - 97°C). The filtrate was concentrated to give 70 g of viscous oil.

The crystalline carbamate was repeatedly recrystallized, first from t-butyl methyl ether, then from methanol and finally from t-butyl methyl ether to give 1,2 g of very pure crystalline α-naphthyl carbamate (mp 117,7° - 117,9°C) which was subjected to X-ray crystallographic analysis.

The X-ray analysis showed that this crystalline carbamate possessed the relative stereochemical configuration at C-2 and C-3 of R* and R* respectively. (See J. Rigaudy and S.P. Klesney, "Nomenclature of Organic Chemistry" 1979 Edition, Pergamon Press, New York, p.482, for naming of chiral centers for which the relative configuration is known.) This diastereomer would therefore be a "syn" or "erythro" diastereomer, namely a syn-(E)-3-methyl-5-(2,2,3--trimethylcyclopent-3-en-1-yl)pent-4-en-2-ol diastereomer. Since the X-ray analysis also determined the relative stereochemical configuration at the third chiral center, ring carbon 1, to be R*, this crystalline carbamate would be the (2R*, 3R*)-(E)-3-methyl-5-(2,2,3-trimethylcyclopent-3-en-1(R*)-yl)pent-4-en-2-ol diastereomer.

In order to isolate and correlate this crystalline carbamate to the component Ia or Ib, the pure solid was subjected to ester hydrolysis and the neutral fraction was isolated. Analysis by $^1$H-NMR and GLC on the ethylene glycol succinate column (LAC-4R-886) showed this neutral fraction to be the third peak (99.5% pure) of I, i.e., the first peak of the component Ib.

In the light of the 400 MHz $^1$H-NMR spectra of Ib which shows that the two diastereomers present in Ib are of similar relative configuration at C-2 and C-3 it can be deduced that the second component of Ib (fourth component of I) would be the other possible syn diastereomer, namely, the (2R*, 3R*)-(E)-3-methyl-5-(2,2,3-trimethylcyclopent-3-en-1(S*)-yl)pent-4-en-2-ol diastereomer.

In order to obtain a crystalline ester of component Ia suitable for X-ray diffraction analysis the viscous oil portion of the α-naphthyl carbamate (80 g) was distilled to separate the volatile material from any residue. The resultant volatile fraction (56 g) was hydrolyzed and the resultant neutral portion (23 g), a mixture of 70 % Ia, 15 % Ib and 15 % impurities, was reacted with 3-nitrophthalic anhydride in the presence of pyridine at 80°C for 1.0 hour. After workup and isolation there was obtained 31 g of crystalline 3-nitrophthalic acid mono ester (mp 118° - 125°C).

The mono ester was repeatedly recrystallized first from a 9 to 1 mixture of hexane/t-butyl methyl ether, then from a 9 to 1 mixture of methanol/water and finally from a mixture of carbon tetrachloride/hexane to give 1.2 g of very pure crystalline 3-nitrophthalic acid mono ester (mp 146.5°C). The crystalline form of this mono ester was not suitable for X-ray diffraction analysis.

The mono ester (1.0 g) was therefore hydrolyzed and the crude product (0.65 g) was distilled at 80°C (0.07 mm) to yield 0.45 g of an oil identified by GLC (LAC-4R-886) as the first peak of I, i.e., the first component of Ia. The oil (0.2 g) was then reacted with ca. 5% solution of isocyanic acid to form the allophonate. (See Helv. 35, 2380 (1952); L. Fieser et al. "Reagens For Organic Synthesis", Vol 1. John Wiley and Sons, Inc. New York, p. 170.) The reaction mixture was left at ambient temperatures for three days in an open flask, extracted with ether and washed with 10% potassium hydroxide solution. Evaporation gave 0.2 g of allophonate crystals which were recrystallized from ethanol to give 0.13 g allophonate (m.p. 154°C). These crystals were suitable for X-ray diffraction analysis.

The X-ray analysis showed that this crystalline allophonate had the relative stereochemical configuration at C-2 and C-3 of R* and S* respectively. This diastereomer would therefore be an "anti" or "threo" diastereomer, namely an anti-(E)-3-methyl-5-(2,2,3-trimethylcyclopent-3-en-1-yl)pent-4-en-2-ol diastereomer. Since the X-ray analysis also determined the relative stereochemical configuration at the third chiral center, ring carbon I, to be S* this crystalline allophonate would be the (2R*, 3S*)-(E)-3-methyl-5-(2,2,3--trimethylcyclopent-3-en-1(S*)-yl)pent-4-en-2-ol diastereomer.

In light of the 400 MHz $^1$H-NMR spectra of Ia which shows that the two diastereomers present in Ia are of similar relative configuration at C-2 and C-3 it can be deduced that the second component of Ia (second component of I) would be the other possible anti diastereomer, namely the (2R*, 3S*)-(E)-3-methyl-5-(2,2,3-trimethyl-3-en-(R*)-yl)-pent-4-en-2-ol diastereomer.

The four isomers as they elute from the GLC (LAC-4R-886) are as follows:

1. (2R*, 3S*)-(E)-3-methyl-5-(2,2,3-trimethylcyclopent-3--en-1(S*)-yl)pent-4-en-2-ol,
2. (2R*, 3S*)-(E)-3-methyl-5-(2,2,3-trimethylcyclopent-3--en-1(R*)-yl)pent-4-en-2-ol,
3. (2R*, 3R*)-(E)-3-methyl-5-(2,2,3-trimethylcyclopent-3--en-1(R*)-yl)pent-4-en-2-ol,and,
4. (2R*, 3R*)-(E)-3-methyl-5-(2,2,3-trimethylcyclopent-3--en-1(S*)-yl)pent-4-en-2-ol.

An odor threshold value comparison of the two diastereomers of Ia indicates that the (2R*, 3S*)-(E)-3-methyl-5--(2,2,3-trimethylcyclopent-3-en-1-(R*)-yl)pent-4-en-2-ol is the stronger of the two diastereomers by a factor of about 10.

Examples 4 and 5

These examples illustrate novel homoallylic alcohols structurally related to 1 [i.e., 2-methyl-4-(2,2,3-trimethylcyclopent-3-en-1-yl)but-3-en-1-ol and 5-(2,2,3-trimethylcyclopent-3-en-1-yl)pent-4-en-2-ol] which were found to possess properties very different than I.

Example 4

Preparation of 2-methyl-4-(2,2,3-trimethylcyclopent-3-en-1-yl)but-3-en-1-ol

The aldehyde, 2-methyl-4-(2,2,3-trimethylcyclopent-3-en-1-yl)but-2-enal, which had been prepared by using the procedure outlined in US-PS 4,052,341 Example IV-1, was reacted essentially following the procedure described in Example 1 for isomerizing the $\alpha,\beta$ double bond to the $\beta,\gamma$ position. In this case the butenal (19,2 g , 0,1 mole) was reacted with potassium t-butoxide (16,8 g, 15 mole) in 1,2-dimethoxyethane (DME, 200 ml) at 15° - 20°C by feeding in the butenal over 30 minutes, stirring for an additional 15 minutes and then rapidly quenching with aqueous 20% acetic acid (75 ml).

After work-up, the crude oil was distilled at reduced pressure to yield 14,6 g of distillate: bp 68-100°C (0,6mm). The distillate was a mixture having the following composition as determined by GC/MS analysis:
29% 2-methyl-4-(2,2,3-trimethylcyclopent-3-en-1-yl)but-3-enal
57% 2-methyl-4-(2,2,3-trimethylcyclopent-3-en-1-yl)but-2-enal (26% Z-isomer, 31% E-isomer)

The above mixture (17,0 g, 88,5 mmoles) of aldehydes was dissolved in anhydrous ethyl ether (25 ml) and was slowly fed into a flask containing a cold (0°C) suspension of lithium aluminium hydride (1,7 g, 44,3 mmole) in anhydrous ether (25 ml).

The resultant mixture was stirred for 30 minutes at 0-5°C, then for 1,0 hour at ambient temperature and then at reflux for 2,0 hours. The mixture was then cooled to 0°C and the excess lithium aluminium hydride was decomposed carefully by the slow, dropwise addition of saturated aqueous sodium sulfate solution.

The resultant suspension was filtered, the ether removed by distillation at atmospheric pressure and the remaining oil distilled under reduced pressure to give 14,0 g of distillate: bp 89-145°C (0,9 mmHg); a mixture of components by GC/MS consisting of:
22% 2-methyl-4-(2,2,3-trimethylcyclopent-3-en-1-yl)but--3-en-1-ol
71% 2-methyl-4-(2,2,3-trimethylcyclopent-3-en-1-yl)but--2-en-1-ol (27% Z-isomer, 44% E-isomer)

This mixture was subjected to a careful spinning band distillation to give:
2-methyl-4-(2,2,3-trimethylcyclopent-3-en-1-yl)but-3-en-1-ol: bp 76,5°C (0,5 mmHg); mol. wt. 194 (MS); IR 3340 cm$^{-1}$ (hydroxyl), 3040, 1670 and 800 (olefinic); Odor Threshold Value 6,0 ng/l; Odor: fruity, woody, anisic.

$^1$H-NMR 0.8$\delta$ (3H, s), 1,0 (3H, s), 1,2 (3H, d, J ~ 6Hz), 1,6 (3H, broad s, olefinic $CH_3$),2,2 and 2,4 (5H, 2 broad complexes), 3,4 (2H, d, J ~ 7Hz), 5,2 (1H, broad multiplet), 5,0 - 5,8 (2H, broad complex olefinic protons).

$^{13}$C-NMR 12,7 ppm (q), 16,8 + 17,0 (2q, C-2 methyl group), 20,5 (q), 25,4 (q), 35,5 (t), 39,7 (d), 47,9 (s), 54,2 (d), 67,5 (t, C-1), 121,5 (d) 132,3 (d), 133,1 + 133,2 (2d,C-3), 147,9 (s). The $^{13}$C-NMR shows 13 distinct carbon resonances with 2 carbon resonances appearing as two resonances indicating the presence of diastereomers. This molecule contains two asymmetric carbon atoms; that is, C-2 of the side-chain and C-1 of the cyclopentenyl ring.

Example 5

Preparation of 5-(2,2,3-trimethylcyclopent-3-en-1-yl)pent-4-en-2-ol

The ketone, 5-(2,2,3-trimethylcyclopent-3-en-1-yl)pent--3-en-2-one, which had been prepared by using the procedure of US-PS 4,052,341, Example II-2, was reacted essentially following the procedure desribed in Example 1 for isomerizing the α,β double bond to the β,γ position. In this case the ketone (28,8 g, 0,15 mole) was reacted with potassium t-butoxide (25,8 g, 0,23 mole) in 1,2-dimethoxyethane (DME) (125 ml). The reaction was quenched by addition of aqueous 20% acetic acid (115 ml), extracted as in Example 1 and the oil distilled at reduced pressure to yield 19,0 g (66% yield) of 5-(2,2,3-trimethylcyclopent-3-en-1-yl)pent--4-en-2-one as a mixture of Z- (21%) and E- (69%) isomers: bp 80-100°C (0,5 mmHg); mol. wt. 192 (MS); IR 3040 $cm^{-1}$ (olefin), 1720 (carbonyl), 800 (trisubstituted olefin).

$^1$H-NMR 0,8δ (3H,s), 1,0 (3H, s), 1,6 (3H, broad s, olefinic $CH_3$), 2,2 (3H, s, acetyl $CH_3$), 1,9-2,7 (3H, broad complex), 3,1 (1H, d, J ~ 6Hz), 3,2 (1H, d, J~6Hz), 5,2 (1H, broad multiplet, ring olefinic $CH_3$), 5,6 (2H, broad multiplet, 2 olefinic H).

$^{13}$C-NMR 12,6 ppm (q), 20,5 (q), 25,4 (q), 29,1 (q), 35,3 (t), 42,5 (s), 48,2 (d), 54,1 (t), 121,5 (d), 122,8 (d), 135,9 (d), 147,8 (s), 206,4 (s, carbonyl). The presence of the Z-isomer as a minor component was indicated by weaker carbon resonances at 20,3 (q), 25,9 (q), 29,3 (q), 36,0 (t), 48,2, 48,7, 121,6 (d), 121,9 (d), 134,6 (d), 205,9 (s, carbonyl).

The ratio of the two carbonyl carbon resonances indicated a 72 % / 28 % mixture, which is in good agreement with the GLC assay.

Using the procedure as outlined in Example 2, 5-(2,2,3--trimethylcyclopent-3-en-1-yl)pent-4-en-2-one (14,4 g, 75 mmoles) was reacted with sodium borohydride powder (1,44 g, 38 mmoles) in a mixture of ethanol (75 ml), water (15 ml) and 30% sodium hydroxide solution (0,25 ml).

The mixture was quenched, worked up and distilled to give 12,4 (85,2 % theory-yield) of 5-(2,2,3-trimethylcyclopent-3-en-1-yl)pent-4-en-2-ol as a mixture of Z- (23%) and E- (69%) isomers: bp 79-81°C (0,5 mmHg); $n_D^{20}$ 1,4820; mol. wt. 194 (MS); IR 3340 $cm^{-1}$ (hydroxyl), 3040 (olefinic), 1655 (olefinic) and 800 (trisubstituted olefin); Odor Threshold Value 720 ng/l; Odor: weak, sandalwood, woody.

$^1$H-NMR 0,7δ (3H, s), 1,0 (3H, s), 1,2 (3H, d, J ~ 6Hz), 1,6 (3H, broad s, olefinic $CH_3$), 2,2 (5H, broad multiplet), 3,1 (1H, broad, exchanges with $D_2O$, hydroxyl H), 3,8 (1H, broad, sextet, J ~ 65Hz), 5,2 (1H, broad multiplet, ring olefinic H), 5,5 (2H, broad complex, 2 olefinic protons).

$^{13}$C-NMR 12,6 ppm) (q), 20,5 (q), 22,6 (q), 25,5 (q) 35,5 (t), 42,8 (s), 47,9 (d), 54,3 (t), 67,4 (d), 121,6 (d), 127,0 (d), 134,2 (d), 147,7 (s). The presence of the Z-isomer as a minor component was indicated by the following carbon resonances: 25,9 (q), 36,4, 37,4, 48,5, 48,6, 67,6 (d), 126,6 (d), 133,3 (d).

Example 6

The following fragrance compositions illustrate the odorant utility of (E)-3-methyl-5-(2,2,3-trimethylcyclopent--3-en-1-yl)pent-4-en-2-ol (I), as prepared in Example 2. In each instance the effects achieved with I could not be achieved with similar amounts of sandalwood oil or 3-methyl--5-(2,2,3-trimethylcyclopent-3-en-1-yl)pentan-2-ol (XII) due to the unique odor intensity of I.

A. Chypre Base

| Component | Parts per weight |
|---|---|
| Bergamot Oil (non-sensitizing; furanocoumarin-free) | 300 |
| Rose Base synthetic (mainly phenyl ethyl alcohol, citronellol, geraniol) | 400 |
| Acetyl Cedrene | 200 |
| Oakmoss Soluble Resin | 60 |
| Vetiver Bourbon | 20 |
| Dipropylene Glycol (DPG) | 15 |
| Total | 995 |

Addition of 5 parts (0.5 %) of I to the chypre base improves the strength and the warm woody quality of the fragrance. (A similar effect can be obtained by using 3 parts of component Ia). In the absence of

the addition of I or Ia, the composition lacks the warm, musky-sandalwood note characteristic of I and Ia. A similar amount of sandalwood oil makes very little contribution to the fragrance.

B. Wood Base

| Component | Parts per weight |
|---|---|
| Sandela® (Givaudan) (Isocamphyl-cyclohexanols) 50% in Diethyl Phthalate | 300 |
| Acetyl Cedrene | 450 |
| Cedrol Crystals | 200 |
| Gamma-Nonalactone | 2 |
| Dipropylene Glycol (DPG) | 25 |
| Total | 977 |

Addition of 25 parts (2.5%) of I to the wood base blends the cedar and the sandalwood notes, and adds body and a warm musk quality to the fragrance, while greatly increasing the strength. (A similar effect can be achieved using 15 parts of Ia in place of I.) Without the addition of I or Ia, the composition is not as full or as strong and lacks the musk note characteristic of I and Ia. A similar amount of sandalwood oil results in a composition that is less intense and less musky.

C. Muguet Base

| Component | Parts per weight |
|---|---|
| Citronellol Synthetic | 200 |
| Phenylethyl Alcohol | 100 |
| Lilial® (Givaudan)(α-methyl-p-tert.-butyl-phenylpropionaldehyde) | 200 |
| Terpineol Synthetic | 50 |
| Linalool | 100 |
| Benzyl Acetate | 100 |
| Hexylcinnamic Aldehyde | 150 |
| Indole, 10% in Dipropylene Glycol (DPG) | 10 |
| Cyclamen Aldehyde (α-methyl-p-iso-propylphenylpropionaldehyde) | 10 |
| Styrax (Natural, non-sensitizing) | 30 |
| Dipropylene Glycol | 47 |
| Total | 997 |

Addition of 3 parts (0.3%) of I to the muguet base blends the individual floral components and adds body, fullness and warmth to the fragrance. (Addition of about 2 parts of Ia in place of I provides a similar effect.) Without the addition of I or Ia the composition lacks unity and fullness. The same effects cannot be achieved with the addition of sandalwood oil or compound XII.

D. Musk Fragrance

| Component | Parts per weight |
|---|---|
| Coumarin | 10 |
| Heliotropin | 35 |
| Vanillin | 2 |
| Benzyl Acetate | 10 |
| Methyl Dihydrojasmonate | 10 |
| Bergamot Synthetic | 40 |
| Dimetol$^R$ (Givaudan) (2,6-dimethyl-heptan-2-ol) | 5 |
| Geranium Oil | 25 |
| Rose Base Synthetic (mainly phenylethyl alcohol, citronellol, geraniol) | 10 |
| Lavender Oil | 25 |
| Lemon Oil, Calfornia | 20 |
| Lilial$^R$ (Givaudan) (α-methyl-p-tert-butylphenylpropionaldehyde) | 10 |
| Oakmoss, Soluble Resin | 5 |
| Folenox$^{TM}$ (Givaudan) (isolongifolene epoxide | 25 |
| Ethylene Brassylate | 200 |
| Fixolide$^R$ (Givaudan) (7-acetyl-1,1,3,4,4,6-hexamethyltetralin) | 200 |
| Amyl Salicylate | 40 |
| Dipropylene Glycol (DPG) | 278 |
| Total | 950 |

Addition of 50 parts (5.0 %) of I to the musk fragrance greatly increases the strength of the fragrance while improving both the intensity and the quality of the musk notes. (A similar effect can be achieved using 30 parts of Ia.) The same effect cannot be achieved using sandalwood oil or compound XII.

**Claims**

1. The two lower boiling diastereomers of (E)-3-methyl-5-(2,2,3-trimethylcyclopent-3-en-1-yl)pent-4-en-2-ol.
2. The two lower boiling diastereomers of (E)-3-methyl-5-(2,2,3-trimethylcyclopent-3-en-1-yl)pent-4-en-2-ol in substantially pure form.
3. A composition comprising at least ten percent of the two lower boiling diastereomers of (E)-3-methyl-5-(2,2,3-trimethylcyclopent-3-en-1-yl)pent-4-en-2-ol.
4. A composition comprising at least twenty-five percent of the two lower boiling diastereomers of (E)-3-methyl-5-(2,2,3-trimethylcyclopent-3-en-1-yl)pent-4-en-2-ol.

5. A composition comprising at least forty percent of the two lower boiling diastereomers of (E)-3-methyl-5-(2,2,3-trimethyl-cyclopent-3-en-1-yl)pent-4-en-2-ol.

6. A composition comprising at least fifty percent of the two lower boiling diastereomers of (E)-3-methyl-5-(2,2,3-trimethyl-cyclopent-3-en-1-yl)pent-4-en-2-ol.

7. A composition comprising at least ninety percent of the two lower boiling diastereomers of (E)-3-methyl-5-(2,2,3-trimethyl-cyclopent-3-en-1-yl)pent-4-en-2-ol.

8. A composition which consists essentially of

a) fifty to one hundred percent of the two lower boiling diastereomers of (E)-3-methyl-5-(2,2,3-trimethylcyclopent-3-en-1-yl)pent-4-en-2-ol,

b) zero to forty-five percent of the two higher boiling diastereomers of (E)-3-methyl-5-(2,2,3-trimethyl-cyclopent-3-en-1-yl)pent-4-en-2-ol,

c) zero to twelve percent of (E)-6-(2,2,3-trimethylcyclopent-3-en-1-yl)hex-5-en-3-ol,

d) zero to ten percent of 3-methyl-5-(2,2,3-trimethyl-cyclopent-3-en-1-yl)pent-3-en-2-ol, and,

e) zero to ten percent of 3-methyl-5-(2,2,3-trimethylcyclopent-3-en-1-yl)pentan-2-ol.

9. A composition which consists essentially of

a) forty to sixty-five percent of the two lower boiling diastereomers of (E)-3-methyl-5-(2,2,3-trimethylcyclopent-3-en-1-yl)pent-4-en-2-ol,

b) twenty-five to forty-five percent of the two higher boiling diastereomers of (E)-3-methyl-5-(2,2,3-trimethylcyclopent-3-en-1-yl)pent-4-en-2-ol,

c) zero to twelve percent of (E)-6-(2,2,3-trimethyl-cyclopent-3-en-1-yl)hex-5-en-3-ol,

d) zero to five percent of 3-methyl-5-(2,2,3-trimethyl-cyclopent-3-en-1-yl)pent-3-en-2-ol, and,

e) zero to five percent of 3-methyl-5-(2,2,3-trimethylcyclopent-3-en-1-yl)pentan-2-ol.

10. A composition according to claim 9, wherein

a) said two lower boiling diastereomers of (E)-3-methyl-5-(2,2,3-trimethylcyclopent-3-en-1-yl)pent-4-en-2-ol are present at a level of forty-five to sixty percent,

b) said two higher boiling diastereomers of (E)-3-methyl-5-(2,2,3-trimethylcyclopent-3-en-1-yl)pent-4-en-2-ol are present at a level of thirty to forty-five percent, and,

c) said (E)-6-(2,2,3-trimethylcyclopent-3-en-1-yl)hex-5-en-3-ol is present at a level of less than ten percent.

11. A fragrance composition to which there have been added as fragrance contributing ingredients the substantially pure two lower boiling diastereomers of (E)-3-methyl-5-(2,2,3-trimethylcyclopent-3-en-1-yl)-pent-4-en-2-ol.

12. A fragrance composition to which there has been added as a fragrance contributing ingredient, a composition comprising at least ten percent of the two lower boiling diastereomers of (E)-3-methyl-5-(2,2,3-trimethylcyclopent-3-en-1-yl)pent-4-en-2-ol.

13. A fragrance composition according to claim 12, wherein the two lower boiling diastereomers of (E)-3-methyl-5-(2,2,3-trimethylcyclopent-3-en-1-yl)pent-4-en-2-ol comprise at least twenty-five percent of said fragrance contributing ingredient added.

14. A fragrance composition according to claim 12, wherein the two lower boiling diastereomers of (E)-3-methyl-5-(2,2,3-trimethylcyclopent-3-en-1-yl)pent-4-en-2-ol comprise at least forty percent of said fragrance contributing ingredient added.

15. A fragrance composition according to claim 12, wherein the two lower boiling diastereomers of (E)-3-methyl-5-(2,2,3-trimethylcyclopent-3-en-1-yl)pent-4-en-2-ol comprise at least fifty percent of said fragrance contributing ingredient added.

16. A fragrance composition according to claim 12, wherein the two lower boiling diastereomers of (E)-3-methyl-5-(2,2,3-trimethylcyclopent-3-en-1-yl)pent-4-en-2-ol comprise at least ninety percent of said fragrance contributing ingredient added.

17. A fragrance composition to which there has been added as a fragrance contributing ingredient, a composition consisting essentially of

a) forty to one hundred percent of the two lower boiling diastereomers (E)-3-methyl-5-(2,2,3-trimethylcyclopent-3-en-1-yl)pent-4-en-2-ol,

b) zero to forty-five percent of the two higher boiling diastereomers of (E)-3-methyl-5-(2,2,3-trimethylcyclopent-3-en-1-yl)pent-4-en-2-ol,

c) zero to twelve percent of (E)-6-(2,2,3-trimethylcyclopent-3-en-1-yl)hex-5-en-3-ol,

d) zero to ten percent of 3-methyl-5-(2,2,3-trimethylcyclopent-3-en-1-yl)pent-3-en-2-ol, and,

e) zero to ten percent of 3-methyl-5-(2,2,3-trimethylcyclopent-3-en-1-yl)pentan-2-ol.

18. A fragrance composition to which there has been added as a fragrance contributing ingredient, a composition consisting essentially of

a) forty to sixty-five percent of the two lower boiling diastereomers of (E)-3-methyl-5-(2,2,3-trimethylcyclopent-3-en-1-yl)pent-4-en-2-ol,

b) twenty-five to forty-five percent of the two higher boiling diastereomers of (E)-3-methyl-5-(2,2,3-trimethylcyclopent-3-en-1-yl)pent-4-en-2-ol,

c) zero to twelve percent of (E)-6-(2,2,3-trimethylcyclopent-3-en-1-yl)hex-5-en-3-ol,

d) zero to five percent of 3-methyl-5-(2,2,3-trimethylcyclopent-3-en-1-yl)pent-3-en-2-ol, and,

e) zero to five percent of 3-methyl-5-(2,2,3-trimethylcyclopent-3-en-1-yl)pentan-2-ol.

19. A fragrance composition according to claim 12, wherein

a) said two lower boiling diastereomers of (E)-3-methyl-5-(2,2,3-trimethylcyclopent-3-en-1-yl)pent-4-en-2-ol are present at a level of forty-five to sixty percent,

b) said two higher boiling diastereomers of (E)-3-methyl-5-(2,2,3-trimethylcyclopent-3-en-1-yl)pent-4-en-2-ol are present at a level of thirty to forty-five percent, and,

c) said (E)-6-(2,2,3-trimethylcyclopent-3-en-1-yl)hex-5-en-3-ol is present at a level of less than ten percent.

20. A fragrance composition according to any one of claims 11 to 19, wherein there is added an amount such that the concentration of the two lower boiling diastereomers of (E)-3-methyl-5-(2,2,3-trimethylcyclopent-3-en-1-yl)pent-4-en-2-ol is between 0,05% and 25% of the total fragrance imparting ingredients.

21. A fragrance composition according to claim 20, wherein there is added an amount such that the concentration of the two lower boiling diastereomers of (E)-3-methyl-5-(2,2,3-trimethylcyclopent-3-en-1-yl)pent-4-en-2-ol is between 0,1% and 10% of the total fragrance imparting ingredients.

22. A process for the preparation of (E)-3-methyl-5-(2,2,3-trimethylcyclopent-3-en-1-yl)pent-4-en-2-ol, which comprises reducing (E)-3-methyl-5-(2,2,3-trimethylcyclopent-3-en-1-yl)pent-4-en-2-one.

23. A process for the preparation of substantially pure (E)-3-methyl-5-(2,2,3-trimethylcyclopent-3-en-1-yl)pent-4-en-2-ol which comprises reducing substantially pure (E)-3-methyl-5-(2,2,3-trimethylcyclopent-3-en-1-yl)pent-4-en-2-one.

24. The use of the substantially pure two lower boiling diastereomers of (E)-3-methyl-5-(2,2,3-trimethylcyclopent-3-en-1-yl)pent-4-en-2-ol as odorants.

## Patentansprüche

1. Die zwei niedriger siedenden Diastereoisomeren von (E)-3-Methyl-5-(2,2,3-trimethylcyclopent-3-en-1-yl)pent-4-en-2-ol.

2. Die zwei niedriger siedenden Diastereoisomeren von (E)-3-Methyl-5-(2,2,3-trimethylcyclopent-3-en-1-yl)pent-4-en-2-ol in im wesentlichen reiner Form.

3. Eine Komposition mit einem Gehalt an wenigstens 10% der zwei niedriger siedenden Diastereomeren von (E)-3-Methyl-5-(2,2,3-trimethylcyclopent-3-en-1-yl)pent-4-en-2-ol.

4. Eine Komposition mit einem Gehalt an wenigstens 25% der zwei niedriger siedenden Diastereomeren von (E)-3-Methyl-5-(2,2,3-trimethylcyclopent-3-en-1-yl)pent-4-en-2-ol.

5. Eine Komposition mit einem Gehalt an wenigstens 40% der zwei niedriger siedenden Diastereomeren von (E)-3-Methyl-5-(2,2,3-trimethyl-cyclopent-3-en-1-yl)pent-4-en-2-ol.

6. Eine Komposition mit einem Gehalt an wenigstens 50% der zwei niedriger siedenden Diastereomeren von (E)-3-Methyl-5-(2,2,3-trimethyl-cyclopent-3-en-1-yl)pent-4-en-2-ol.

7. Eine Komposition mit einem Gehalt an wenigstens 90% der zwei niedriger siedenden Diastereomeren von (E)-3-Methyl-5-(2,2,3-trimethyl-cyclopent-3-en-1-yl)pent-4-en-2-ol.

8. Eine Komposition, enthaltend im wesentlichen

a) 50 bis 100% der zwei niedriger siedenden Diastereomeren von (E)-3-Methyl-5-(2,2,3-trimethylcyclopent-3-en-1-yl)pent-4-en-2-ol,

b) 0 bis 45% der zwei höher siedenden Diastereomeren von (E)-3-Methyl-5-(2,2,3-trimethyl-cyclopent-3-en-1-yl)pent-4-en-2-ol,

c) 0 bis 12% von (E)-6-(2,2,3-Trimethylcyclopent-3-en-1-yl)hex-5-en-3-ol,

d) 0 bis 10% von 3-Methyl-5-(2,2,3-trimethyl-cyclopent-3-en-1-yl)pent-3-en-2-ol, und

e) 0 bis 10% von 3-Methyl-5-(2,2,3-trimethylcyclopent-3-en-1-yl)pentan-2-ol.

9. Eine Komposition, enthaltend im wesentlichen

a) 40 bis 65% der zwei niedriger siedenden Diastereomeren von (E)-3-Methyl-5-(2,2,3-trimethyl-cyclopent-3-en-1-yl)pent-4-en-2-ol,

b) 25 bis 45% der zwei höher siedenden Diastereomeren von (E)-3-Methyl-5-(2,2,3-trimethylcyclopent-3-en-1-yl)pent-4-en-2-ol,

c) 0 bis 12% von (E)-6-(2,2,3-Trimethyl-cyclopent-3-en-1-yl)hex-5-en-3-ol,

d) 0 bis 5% von 3-Methyl-5-(2,2,3-trimethyl-cyclopent-3-en-1-yl)pent-3-en-2-ol, und

e) 0 bis 5% von 3-Methyl-5-(2,2,3-trimethylcyclopent-3-en-1-yl)pentan-2-ol.

10. Eine Komposition gemäß Anspruch 9, worin

a) der Gehalt an den zwei niedriger siedenden Diastereomeren von (E)-3-Methyl-5-(2,2,3-trimethylcyclopent-3-en-1-yl)pent-4-en-2-ol 45 bis 60% beträgt,

b) der Gehalt an den zwei höher siedenden Diastereomeren von (E)-3-Methyl-5-(2,2,3-trimethylcyclopent-3-en-1-yl)pent-4-en-2-ol 30 bis 45% beträgt, und

c) (E)-6-(2,2,3-Trimethylcyclopent-3-en-1-yl)hex-5-en-3-ol zu weniger als 10% enthalten ist.

11. Riechstoffkomposition, zu welcher als riechende Komponenten die zwei niedriger siedenden Diastereomeren von (E)-3-Methyl-5-(2,2,3-trimethylcyclopent-3-en-1-yl)pent-4-en-2-ol in im wesentlichen reiner Form zugegeben wurden.

12. Riechstoffkomposition, zu welcher als riechende Komponente eine Komposition mit einem Gehalt an wenigstens 10% der zwei niedriger siedenden Diastereomeren von (E)-3-Methyl-5-(2,2,3-trimethylcyclopent-3-en-1-yl)pent-4-en-2-ol zugegeben wurde.

13. Riechstoffkomposition gemäß Anspruch 12, worin die zwei niedriger siedenden Diastereomeren von (E)-3-Methyl-5-(2,2,3-trimethylcyclopent-3-en-1-yl)pent-4-en-2-ol wenigstens 25% der zugegebenen riechenden Komponente betragen.

14. Riechstoffkomposition gemäß Anspruch 12, worin die zwei niedriger siedenden Diastereomeren von (E)-3-Methyl-5-(2,2,3-trimethylcyclopent-3-en-1-yl)pent-4-en-2-ol wenigstens 40% der zugegebenen riechenden Komponente betragen.

15. Eine Riechstoffkomposition gemäß Anspruch 12, worin die zwei niedriger siedenden Diastereomeren von (E)-3-Methyl-5-(2,2,3-trimethylcyclopent-3-en-1-yl)pent-4-en-2-ol wenigstens 50% der zugegebenen riechenden Komponente betragen.

16. Eine Riechstoffkomposition gemäß Anspruch 12, worin die zwei niedriger siedenden Diastereomeren von (E)-3-Methyl-5-(2,2,3-trimethylcyclopent-3-en-1-yl)pent-4-en-2-ol wenigstens 90% der zugegebenen riechenden Komponente betragen.

17. Eine Riechstoffkomposition, zu welcher als riechende Komponente eine Komposition zugegeben wurde, welche im wesentlichen aus

a) 40 bis 100% der zwei niedriger siedenden Diastereomeren von (E)-3-Methyl-5-(2,2,3-trimethylcyclopent-3-en-1-yl)pent-4-en-2-ol,

b) 0 bis 45% der zwei höher siedenden Diastereomeren von (E)-3-Methyl-5-(2,2,3-trimethylcyclopent-3-en-1-yl)pent-4-en-2-ol,

c) 0 bis 12% (E)-6-(2,2,3-Trimethylcyclopent-3-en-1-yl)hex-5-en-3-ol,

d) 0 bis 10% 3-Methyl-5-(2,2,3-trimethylcyclopent-3-en-1-yl)pent-3-en-2-ol, und

d) 0 bis 10% 3-Methyl-5-(2,2,3-trimethylcyclopent-3-en-1-yl)pentan-2-ol

besteht.

18. Riechstoffkomposition, zu welcher als riechende Komponente eine Komposition zugegeben wurde, welche im wesentlichen aus

a) 40 bis 65% der zwei niedriger siedenden Diastereomeren von (E)-3-Methyl-5-(2,2,3-trimethylcyclopent-3-en-1-yl)pent-4-en-2-ol,

b) 25 bis 45% der zwei höher siedenden Diastereomeren von (E)-3-Methyl-5-(2,2,3-trimethylcyclopent-4-en-2-ol,

c) 0 bis 12% (E)-6-(2,2,3-Trimethylcyclopent-3-en-1-yl)hex-5-en-ol,

d) 0 bis 5% 3-Methyl-5-(2,2,3-trimethylcyclopent-3-en-1-yl)pent-3-en-2-ol, und,

e) 0 bis 5% 3-Methyl-5-(2,2,3-trimethylcyclopent-3-en-1-yl)pentan-2-ol

besteht.

19. Riechstoffkomposition gemäß Anspruch 12, worin

a) die zwei niedriger siedenden Diastereomeren von (E)-3-Methyl-5-(2,2,3-trimethylcyclopent-3-en-1-yl)pent-4-en-2-ol 45 bis 60% ausmachen,

b) die zwei höher siedenden Diastereomeren von (E)-3-Methyl-5-(2,2,3-trimethylcyclopent-3-en-1-yl)pent-4-en-2-ol 30 bis 45% ausmachen, und,

c) (E)-6-(2,2,3-Trimethylcyclopent-3-en-1-yl)hex-5-en-3-ol weniger als 10% ausmacht.

20. Riechstoffkomposition gemäß einem der Ansprüche 11 bis 19, worin eine solche Menge zugegeben wurde, daß die Konzentration an den zwei niedriger siedenden Diastereomeren von (E)-3-Methyl-5-(2,2,3-trimethylcyclopent-3-en-1-yl)pent-4-en-2-ol zwischen 0,05% und 25% der gesamten riechenden Komponente beträgt.

21. Riechstoffkomposition gemäß Anspruch 20, worin eine solche Menge zugegeben wurde, daß die Konzentration der zwei niedriger siedenden Diastereomeren von (E)-3-Methyl-5-(2,2,3-trimethylcyclopent-3-en-1-yl)pent-4-en-2-ol zwischen 0,1% und 10% der gesamten riechenden Komponente beträgt.

22. Verfahren zur Herstellung von (E)-3-Methyl-5-(2,2,3-trimethylcyclopent-3-en-1-yl)pent-4-en-2-ol, dadurch gekennzeichnet, daß man (E)-3-Methyl-5-(2,2,3-trimethylcyclopent-3-en-1-yl)pent-4-en-2-on reduziert.

23. Verfahren zur Herstellung von im wesentlichen reinem (E)-3-Methyl-5-(2,2,3-trimethylcyclopent-3-en-1-yl)pent-4-en-2-ol, dadurch gekennzeichnet, daß man im wesentlichen reines (E)-3-Methyl-5-(2,2,3-trimethylcyclopent-3-en-1-yl)pent-4-en-2-on reduziert.

24. Verwendung der im wesentlichen reinen zwei niedriger siedenden Diastereomeren von (E)-3-Methyl-5-(2,2,3-trimethylcyclopent-3-en-1-yl)pent-4-en-2-ol als Riechstoffe.

## Revendications

1. Les deux diastéréoisomères, de point d'ébullition plus faible, du (E)-3-méthyl-5-(2,2,3-triméthylcyclopent-3-en-1-yl)-pent-4-en-2-ol.

2. Les deux diastéréoisomères, de point d'ébullition plus faible, du (E)-3-méthyl-5-(2,2,3-triméthylcyclopent-3-en-1-yl)-pent-4-en-2-ol, sous une forme pratiquement pure.

3. Composition comprenant au moins dix pour-cent des deux diastéréoisomères, de point d'ébullition plus faible, du (E)-3-méthyl-5-(2,2,3-triméthylcyclopent-3-en-1-yl)-pent-4-en-2-ol.

4. Composition comprenant au moins vingt cinq pour-cent des deux diastéréoisomères, de point d'ébullition plus faible, du (E)-3-méthyl-5-(2,2,3-triméthylcyclopent-3-en-1-yl)-pent-4-en-2-ol.

5. Composition comprenant au moins quarante pour-cent des deux diastéréoisomères, de point d'ébullition plus faible, du (E)-3-méthyl-5-(2,2,3-triméthylcyclopent-3-en-1-yl)-pent-4-en-2-ol.

6. Composition comprenant au moins cinquante pour-cent des deux diastéréoisomères, de point d'ébullition plus faible, du (E)-3-méthyl-5-(2,2,3-méthylcyclopent-3-en-1-yl)-pent-4-en-2-ol.

7. Composition comprenant au moins quatre vingt dix pour-cent des deux diastéréoisomères, de point d'ébullition plus faible, du (E)-3-méthyl-5-(2,2,3-triméthylcyclopent-3-en-1-yl)-pent-4-en-2-ol.

8. Composition qui comprend essentiellement:

a) cinquante à cent pour-cent des deux diastéréoisomères, de point d'ébullition plus faible, du (E)-3-méthyl-5-(2,2,3-triméthylcyclopent-3-en-1-yl)-pent-4-en-2-ol,

b) zéro à quarante cinq pour-cent des deux diastéréoisomères, de point d'ébullition plus élevé, du (E)-3-méthyl-5-(2,2,3-triméthylcyclopent-3-en-1-yl)-pent-4-en-2-ol,

c) zéro à douze pour-cent de (E)-6-(2,2,3-triméthylcyclopent-3-en-1-yl)hex-5-en-3-ol,

d) zéro à dix pour-cent de 3-méthyl-5-(2,2,3-triméthylcyclopent-3-en-1-yl)pent-3-en-2-ol, et

e) zéro à dix pour-cent de 3-méthyl-5-(2,2,3-triméthylcyclopent-3-en-1-yl)pentan-2-ol.

9. Composition qui comprend essentiellement:

a) quarante à soixante cinq pour-cent des deux diastéréoisomères, de point d'ébullition plus faible, du (E)-3-méthyl-5-(2,2,3-triméthylcyclopent-3-en-1-yl)-pent-4-en-2-ol,

b) vingt cinq à quarante cinq pour-cent des deux diastéréoisomères, de point d'ébullition plus élevé, du (E)-3-méthyl-5-(2,2,3-triméthylcyclopent-3-en-1-yl)-pent-4-en-2-ol,

c) zéro à douze pour-cent de (E)-6-(2,2,3-triméthylcyclopent-3-en-1-yl)-hex-5-en-3-ol,

d) zéro à cinq pour-cent de 3-méthyl-5-(2,2,3-triméthylcyclopent-3-en-1-yl)pent-3-en-2-ol, et

e) zéro à cinq pour-cent de 3-méthyl-5-(2,2,3-triméthylcyclopent-3-en-1-yl)pentan-2-ol.

10. Composition selon la revendication 9, dans laquelle

a) lesdits deux diastéréoisomères, de point d'ébullition plus faible, du (E)-3-méthyl-5-(2,2,3-triméthylcyclopent-3-en-1-yl)-pent-4-en-2-ol sont présents à un taux de quarante cinq à soixante pour-cent,

b) lesdits deux diastéréoisomères, de point d'ébullition plus élevé, du (E)-3-méthyl-5-(2,2,3-triméthylcyclopent-3-en-1-yl)-pent-4-en-2-ol sont présents à un taux de trente à quarante cinq pour-cent, et

c) ledit (E)-6-(2,2,3-triméthylcyclopent-3-en-1-yl)-hex-5-en-3-ol est présent à un taux de moins de dix pour-cent.

11. Composition de fragrance à laquelle on a ajouté, à titre d'ingrédients contribuant à la fragrance, les deux diastéréoisomères, pratiquement purs, de point d'ébullition plus faible, du (E)-3-méthyl-5-(2,2,3-triméthylcyclopent-3-en-1-yl)-pent-4-en-2-ol.

12. Composition de fragrance à laquelle on a ajouté, à titre d'ingrédient contribuant à la fragrance, une composition comprenant au moins dix pour-cent des deux diastéréoisomères, de point d'ébullition plus faible, du (E)-3-méthyl-5-(2,2,3-triméthylcyclopent-3-en-1-yl)-pent-4-en-2-ol.

13. Composition de fragrance selon la revendication 12, dans laquelle les deux diastéréoisomères, de point d'ébullition plus faible, du (E)-3-méthyl-5-(2,2,3-triméthylcyclopent-3-en-1-yl)-pent-4-en-2-ol englobent au moins vingt cinq pour-cent dudit ingrédient ajouté, contribuant à la fragrance.

14. Composition de fragrance selon la revendication 12, dans laquelle les deux diastéréoisomères, de point d'ébullition plus faible, du (E)-3-méthyl-5-(2,2,3-triméthylcyclopent-3-en-1-yl)-pent-4-en-2-ol englobent au moins quarante pour-cent dudit ingrédient ajouté, contribuant à la fragrance.

15. Composition de fragrance selon la revendication 12, dans laquelle les deux diastéréoisomères, de point d'ébullition plus faible, du (E)-3-méthyl-5-(2,2,3-triméthylcyclopent-3-en-1-yl)pent-4-en-2-ol englobent au moins cinquante pour-cent dudit ingrédient ajouté, contribuant à la fragrance.

16. Composition de fragrance selon la revendication 12, dans laquelle les deux diastéréoisomères, de point d'ébullition plus faible, du (E)-méthyl-5-(2,2,3-triméthylcyclopent-3-en-1-yl)-pent-4-en-2-ol englobent au moins quatre vingt dix pour-cent dudit ingrédient ajouté, contribuant à la fragrance.

17. Composition de fragrance à laquelle on a ajouté, à titre d'ingrédient contribuant à la fragrance, une composition comprenant essentiellement

a) quarante à cent pour-cent des deux diastéréoisomères, de point d'ébullition plus faible, du (E)-3-méthyl-5-(2,2,3-triméthyl-cyclopent-3-en-1-yl)pent-4-en-2-ol,

b) zéro à quarante cinq pour-cent des deux diastéréoisomères, de point d'ébullition plus élevé, du (E)-3-méthyl-5-(2,2,3-triméthylcyclopent-3-en-1-yl)pent-4-en-2-ol,

c) zéro à douze pour-cent de (E)-6-(2,2,3-triméthylcyclopent-3-en-1-yl)hex-5-en-3-ol,

d) zéro à dix pour-cent de 3-méthyl-5-(2,2,3-triméthylcyclopent-3-en-1-yl)pent-3-en-2-ol, et

e) zéro à dix pour-cent de 3-méthyl-5-(2,2,3-triméthylcyclopent-3-en-1-yl)pentan-2-ol.

18. Composition de fragrance à laquelle on a ajouté, à titre d'ingrédient contribuant à la fragrance, une composition comprenant essentiellement

a) quarante à soixante cinq pour-cent des deux diastéréoisomères, de point d'ébullition plus faible, du (E)-3-méthyl-5-(2,2,3-triméthylcyclopent-3-en-1-yl)pent-4-en-2-ol,

b) vingt cinq à quarante cinq pour-cent des deux diastéréoisomères, de point d'ébullition plus élevé, du (E)-3-méthyl-5-(2,2,3-triméthylcyclopent-3-en-1-yl)pent-4-en-2-ol,

c) zéro à douze pour-cent de (E)-6-(2,2,3-triméthylcyclopent-3-en-1-yl)hex-5-en-3-ol,

d) zéro à cinq pour-cent de 3-méthyl-5-(2,2,3-triméthylcyclopent-3-en-1-yl)pent-3-en-2-ol, et

e) zéro à cinq pour-cent de 3-méthyl-5-(2,2,3-triméthylcyclopent-3-en-1-yl)pentan-2-ol.

19. Composition de fragrance selon la revendication 12, dans laquelle
a) lesdits deux diastéréoisomères, de point d'ébullition plus faible, du (E)-3-méthyl-5-(2,2,3-triméthylcyclopent-3-en-1-yl)-pent-4-en-2-ol sont présents à un taux de quarante cinq à soixante pour-cent,
b) lesdits deux diastéréoisomères, de point d'ébullition plus élevé, du (E)-3-méthyl-5-(2,2,3-triméthylcyclopent-3-en-1-yl)-pent-4-en-2-ol sont présents à un taux de trente à quarante cinq pour-cent, et
c) ledit (E)-6-(2,2,3-triméthylcyclopent-3-en-1-yl)-hex-5-en-3-ol est présent à un taux de moins de dix pour-cent.

20. Composition de fragrance selon l'une quelconque des revendications 11 à 19, dans laquelle on ajoute une quantité telle que la concentration des deux diastéréoisomères, de point d'ébullition plus faible, du (E)-3-méthyl-5-(2,2,3-triméthylcyclopent-3-en-1-yl)pent-4-en-2-ol soit comprise entre 0,05 pour-cent et vingt cinq pour-cent des ingrédients totaux conférant la fragrance.

21. Composition de fragrance selon la revendication 20, dans laquelle on ajoute une quantité telle que la concentration des deux diastéréoisomères, de point d'ébullition plus faible, du (E)-3-méthyl-5-(2,2,3-triméthylcyclopent-3-en-1-yl)pent-4-en-2-ol soit comprise entre 0,1 pour-cent et dix pour-cent des ingrédients totaux conférant la fragrance.

22. Procédé pour la préparation de (E)-3-méthyl-5-(2,2,3-triméthylcyclopent-3-en-1-yl)pent-4-en-2-ol, qui comprend la réduction de la (E)-3-méthyl-5-(2,2,3-triméthylcyclopent-3-en-1-yl)pent-4-en-2-one.

23. Procédé pour la préparation de (E)-3-méthyl-5-(2,2,3-triméthylcyclopent-3-en-1-yl)pent-4-en-2-ol pratiquement pur, qui comprend la réduction de la (E)-3-méthyl-5-(2,2,3-triméthylcyclopent-3-en-1-yl)pent-4-en-2-one pratiquement pure.

24. Utilisation des deux diastéréoisomères pratiquement purs, de point d'ébullition plus faible, du (E)-3-méthyl-5-(2,2,3-triméthylcyclopent-3-en-1-yl)pent-4-en-2-ol, à titre d'agents parfumants.